# EUROPEAN PATENT APPLICATION

(11) **EP 2 653 529 A1**
(43) Date of publication of application: **23.10.2013**
(21) Application number: 11848427.8
(22) Date of filing: 19.12.2011
(51) Int. Cl.: C12M 1/00, B01J 19/00, C07B 61/00, C07C 51/38, C07C 53/02, C07C 59/01, C12P 7/40, C12P 7/42, C12P 7/46, C12N 15/09

(54) **SYNTHETIC PATHWAY CONSTRUCTING EQUIPMENT, SYNTHETIC PATHWAY CONSTRUCTING METHOD, SYNTHETIC PATHWAY CONSTRUCTING PROGRAM, AND PROCESSES FOR MANUFACTURING 3-HYDROXYPROPIONIC ACID, CROTONYL ALCOHOL AND BUTADIENE**

(30) Priority: 17.12.2010 JP 2010281794; 19.08.2011 JP 2011179573
(71) Applicant: Mitsubishi Chemical Corporation, Chiyoda-ku Tokyo 100-8251 (JP)
(72) Inventor: ARAKI, Michihiro, Sakyo-ku Kyoto-shi, Kyoto 606-8501 (JP); MIYAOKU, Kohei, Yokohama-shi, Kanagawa 227-8502 (JP); TANIGUCHI, Takeshi, Yokohama-shi, Kanagawa 227-8502 (JP)
(74) Representative: Vossius & Partner
(86) International application number: PCT/JP2011/079378
(87) International publication number: WO 2012/081723

(57) **Abstract**

A compound synthetic pathway is generated without limiting chemical conversion rules. A synthetic pathway generating apparatus includes: a converting unit to convert chemical structures of compounds into numerical values; a calculation unit to create a compound pair and to calculate a difference value by subtracting the numerical value of one compound of the compound pair from the numerical value of the other compound thereof; a determining unit to compare the difference value of the compound pair of the reaction between which is already known with the difference value of the compound pair of the reaction between which is unknown, and to determine unknown reactions of the compound pairs with the difference values being coincident as virtual reactions; a generating unit to generate compound synthetic pathways by use of nodes representing the compounds and edges representing the known reaction and the virtual reaction respectively; and a selecting unit to select a synthetic pathway from a starting compound to a target compound out of the generated compound synthetic pathways.

SEQUENCE LISTING

<110>  Mitsubishi Chemical Corporation

<120>  SYNTHETIC PATHWAY GENERATING APPARATUS, SYNTHESIS PATHWAY
GENERATING METHOD, SYNTHESIS PATHWAY GENERATING PROGRAM, AND METHOD
OF PRODUCING 3-HYDROXYPROPIONIC ACID, CROTONYL ALCOHOL AND BUTADIENE

<130>  B11111-11359

<150>  JP2010-281794
<151>  2010-12-17

<150>  JP2011-179573
<151>  2011-08-19

<160>  4

<170>  PatentIn version 3.3

<210>  1
<211>  2802
<212>  DNA
<213>  Escherichia coli

<220>
<221>  CDS
<222>  (1)..(2799)

<400>  1
atg cag aac agc gct ttg aaa gcc tgg ttg gac tct tct tac ctc tct      48
Met Gln Asn Ser Ala Leu Lys Ala Trp Leu Asp Ser Ser Tyr Leu Ser
1               5                   10                  15
ggc gca aac cag agc tgg ata gaa cag ctc tat gaa gac ttc tta acc      96
Gly Ala Asn Gln Ser Trp Ile Glu Gln Leu Tyr Glu Asp Phe Leu Thr
            20                  25                  30
gat cct gac tcg gtt gac gct aac tgg cgt tcg acg ttc cag cag tta      144
Asp Pro Asp Ser Val Asp Ala Asn Trp Arg Ser Thr Phe Gln Gln Leu
        35                  40                  45
cct ggt acg gga gtc aaa ccg gat caa ttc cac tct caa acg cgt gaa      192
Pro Gly Thr Gly Val Lys Pro Asp Gln Phe His Ser Gln Thr Arg Glu
    50                  55                  60
tat ttc cgc cgc ctg gcg aaa gac gct tca cgt tac tct tca acg atc      240
Tyr Phe Arg Arg Leu Ala Lys Asp Ala Ser Arg Tyr Ser Ser Thr Ile
65                  70                  75                  80
tcc gac cct gac acc aat gtg aag cag gtt aaa gtc ctg cag ctc att      288
Ser Asp Pro Asp Thr Asn Val Lys Gln Val Lys Val Leu Gln Leu Ile
                85                  90                  95
aac gca tac cgc ttc cgt ggt cac cag cat gcg aat ctc gat ccg ctg      336
Asn Ala Tyr Arg Phe Arg Gly His Gln His Ala Asn Leu Asp Pro Leu
            100                 105                 110
gga ctg tgg cag caa gat aaa gtg gcc gat ctg gat ccg tct ttc cac      384
Gly Leu Trp Gln Gln Asp Lys Val Ala Asp Leu Asp Pro Ser Phe His
        115                 120                 125
gat ctg acc gaa gca gac ttc cag gag acc ttc aac gtc ggt tca ttt      432
Asp Leu Thr Glu Ala Asp Phe Gln Glu Thr Phe Asn Val Gly Ser Phe

130                    135                    140
gcc agc ggc aaa gaa acc atg aaa ctc ggc gag ctg ctg gaa gcc ctc          480
Ala Ser Gly Lys Glu Thr Met Lys Leu Gly Glu Leu Leu Glu Ala Leu
145                    150                    155                    160
aag caa acc tac tgc ggc ccg att ggt gcc gag tat atg cac att acc          528
Lys Gln Thr Tyr Cys Gly Pro Ile Gly Ala Glu Tyr Met His Ile Thr
                165                    170                    175
agc acc gaa gaa aaa cgc tgg atc caa cag cgt atc gag tct ggt cgc          576
Ser Thr Glu Glu Lys Arg Trp Ile Gln Gln Arg Ile Glu Ser Gly Arg
            180                    185                    190
gcg act ttc aat agc gaa gag aaa aaa cgc ttc tta agc gaa ctg acc          624
Ala Thr Phe Asn Ser Glu Glu Lys Lys Arg Phe Leu Ser Glu Leu Thr
        195                    200                    205
gcc gct gaa ggt ctt gaa cgt tac ctc ggc gca aaa ttc cct ggc gca          672
Ala Ala Glu Gly Leu Glu Arg Tyr Leu Gly Ala Lys Phe Pro Gly Ala
    210                    215                    220
aaa cgc ttc tcg ctg gaa ggc ggt gac gcg tta atc ccg atg ctt aaa          720
Lys Arg Phe Ser Leu Glu Gly Gly Asp Ala Leu Ile Pro Met Leu Lys
225                    230                    235                    240
gag atg atc cgc cac gct ggc aac agc ggc acc cgc gaa gtg gtt ctc          768
Glu Met Ile Arg His Ala Gly Asn Ser Gly Thr Arg Glu Val Val Leu
                245                    250                    255
ggg atg gcg cac cgt ggt cgt ctg aac gtg ctg gtg aac gtg ctg ggt          816
Gly Met Ala His Arg Gly Arg Leu Asn Val Leu Val Asn Val Leu Gly
            260                    265                    270
aaa aaa ccg caa gac ttg ttc gac gag ttc gcc ggt aaa cat aaa gaa          864
Lys Lys Pro Gln Asp Leu Phe Asp Glu Phe Ala Gly Lys His Lys Glu
        275                    280                    285
cac ctc ggc acg ggt gac gtg aaa tac cac atg ggc ttc tcg tct gac          912
His Leu Gly Thr Gly Asp Val Lys Tyr His Met Gly Phe Ser Ser Asp
    290                    295                    300
ttc cag acc gat ggc ggc ctg gtg cac ctg gcg ctg gcg ttt aac ccg          960
Phe Gln Thr Asp Gly Gly Leu Val His Leu Ala Leu Ala Phe Asn Pro
305                    310                    315                    320
tct cac ctt gag att gta agc ccg gta gtt atc ggt tct gtt cgt gcc          1008
Ser His Leu Glu Ile Val Ser Pro Val Val Ile Gly Ser Val Arg Ala
                325                    330                    335
cgt ctg gac aga ctt gat gag ccg agc agc aac aaa gtg ctg cca atc          1056
Arg Leu Asp Arg Leu Asp Glu Pro Ser Ser Asn Lys Val Leu Pro Ile
            340                    345                    350
acc atc cac ggt gac gcc gca gtg acc ggg cag ggc gtg gtt cag gaa          1104
Thr Ile His Gly Asp Ala Ala Val Thr Gly Gln Gly Val Val Gln Glu
        355                    360                    365
acc ctg aac atg tcg aaa gcg cgt ggt tat gaa gtt ggc ggt acg gta          1152
Thr Leu Asn Met Ser Lys Ala Arg Gly Tyr Glu Val Gly Gly Thr Val
    370                    375                    380
cgt atc gtt atc aac aac cag gtt ggt ttc acc acc tct aat ccg ctg          1200
Arg Ile Val Ile Asn Asn Gln Val Gly Phe Thr Thr Ser Asn Pro Leu
385                    390                    395                    400
gat gcc cgt tct acg ccg tac tgt act gat atc ggt aag atg gtt cag          1248
Asp Ala Arg Ser Thr Pro Tyr Cys Thr Asp Ile Gly Lys Met Val Gln
                405                    410                    415
gcc ccg att ttc cac gtt aac gcg gac gat ccg gaa gcc gtt gcc ttt          1296
Ala Pro Ile Phe His Val Asn Ala Asp Asp Pro Glu Ala Val Ala Phe
            420                    425                    430
gtg acc cgt ctg gcg ctc gat ttc cgt aac acc ttt aaa cgt gat gtc          1344
Val Thr Arg Leu Ala Leu Asp Phe Arg Asn Thr Phe Lys Arg Asp Val
        435                    440                    445

ttc atc gac ctg gtg tgc tac cgc cgt cac ggc cac aac gaa gcc gac 1392
Phe Ile Asp Leu Val Cys Tyr Arg Arg His Gly His Asn Glu Ala Asp
    450                 455                 460
gag ccg agc gca acc cag ccg ctg atg tat cag aaa atc aaa aaa cat 1440
Glu Pro Ser Ala Thr Gln Pro Leu Met Tyr Gln Lys Ile Lys Lys His
465                 470                 475                 480
ccg aca ccg cgc aaa atc tac gct gac aag ctg gag cag gaa aaa gtg 1488
Pro Thr Pro Arg Lys Ile Tyr Ala Asp Lys Leu Glu Gln Glu Lys Val
                485                 490                 495
gcg acg ctg gaa gat gcc acc gag atg gtt aac ctg tac cgc gat gcg 1536
Ala Thr Leu Glu Asp Ala Thr Glu Met Val Asn Leu Tyr Arg Asp Ala
            500                 505                 510
ctg gat gct ggc gat tgc gta gtg gca gag tgg cgt ccg atg aac atg 1584
Leu Asp Ala Gly Asp Cys Val Val Ala Glu Trp Arg Pro Met Asn Met
        515                 520                 525
cac tct ttc acc tgg tcg ccg tac ctc aac cac gaa tgg gac gaa gag 1632
His Ser Phe Thr Trp Ser Pro Tyr Leu Asn His Glu Trp Asp Glu Glu
    530                 535                 540
tac ccg aac aaa gtt gag atg aag cgc ctg cag gag ctg gcg aaa cgc 1680
Tyr Pro Asn Lys Val Glu Met Lys Arg Leu Gln Glu Leu Ala Lys Arg
545                 550                 555                 560
atc agc acg gtg ccg gaa gca gtt gaa atg cag tct cgc gtt gcc aag 1728
Ile Ser Thr Val Pro Glu Ala Val Glu Met Gln Ser Arg Val Ala Lys
                565                 570                 575
att tat ggc gat cgc cag gcg atg gct gcc ggt gag aaa ctg ttc gac 1776
Ile Tyr Gly Asp Arg Gln Ala Met Ala Ala Gly Glu Lys Leu Phe Asp
            580                 585                 590
tgg ggc ggt gcg gaa aac ctc gct tac gcc acg ctg gtt gat gaa ggc 1824
Trp Gly Gly Ala Glu Asn Leu Ala Tyr Ala Thr Leu Val Asp Glu Gly
        595                 600                 605
att ccg gtt cgc ctg tcg ggt gaa gac tcc ggt cgc ggt acc ttc ttc 1872
Ile Pro Val Arg Leu Ser Gly Glu Asp Ser Gly Arg Gly Thr Phe Phe
    610                 615                 620
cac cgc cac gcg gtg atc cac aac cag tct aac ggt tcc act tac acg 1920
His Arg His Ala Val Ile His Asn Gln Ser Asn Gly Ser Thr Tyr Thr
625                 630                 635                 640
ccg ctg caa cat atc cat aac ggg cag ggc gcg ttc cgt gtc tgg gac 1968
Pro Leu Gln His Ile His Asn Gly Gln Gly Ala Phe Arg Val Trp Asp
                645                 650                 655
tcc gta ctg tct gaa gaa gca gtg ctg gcg ttt gaa tat ggt tat gcc 2016
Ser Val Leu Ser Glu Glu Ala Val Leu Ala Phe Glu Tyr Gly Tyr Ala
            660                 665                 670
acc gca gaa cca cgc act ctg acc atc tgg gaa gcg cag ttc ggt gac 2064
Thr Ala Glu Pro Arg Thr Leu Thr Ile Trp Glu Ala Gln Phe Gly Asp
        675                 680                 685
ttc gcc aac ggt gcg cag gtg gtt atc gac cag ttc atc tcc tct ggc 2112
Phe Ala Asn Gly Ala Gln Val Val Ile Asp Gln Phe Ile Ser Ser Gly
    690                 695                 700
gaa cag aaa tgg ggc cgg atg tgt ggt ctg gtg atg ttg ctg ccg cac 2160
Glu Gln Lys Trp Gly Arg Met Cys Gly Leu Val Met Leu Leu Pro His
705                 710                 715                 720
ggt tac gaa ggg cag ggg ccg gag cac tcc tcc gcg cgt ctg gaa cgt 2208
Gly Tyr Glu Gly Gln Gly Pro Glu His Ser Ser Ala Arg Leu Glu Arg
                725                 730                 735
tat ctg caa ctt tgt gct gag caa aac atg cag gtt tgc gta ccg tct 2256
Tyr Leu Gln Leu Cys Ala Glu Gln Asn Met Gln Val Cys Val Pro Ser
            740                 745                 750
acc ccg gca cag gtt tac cac atg ctg cgt cgt cag gcg ctg cgc ggg 2304

Thr Pro Ala Gln Val Tyr His Met Leu Arg Arg Gln Ala Leu Arg Gly
        755                 760                 765
atg cgt cgt ccg ctg gtc gtg atg tcg ccg aaa tcc ctg ctg cgt cat 2352
Met Arg Arg Pro Leu Val Val Met Ser Pro Lys Ser Leu Leu Arg His
    770                 775                 780
ccg ctg gcg gtt tcc agc ctc gaa gaa ctg gcg aac ggc acc ttc ctg 2400
Pro Leu Ala Val Ser Ser Leu Glu Glu Leu Ala Asn Gly Thr Phe Leu
785                 790                 795                 800
cca gcc atc ggt gaa atc gac gag ctt gat ccg aag ggc gtg aag cgc 2448
Pro Ala Ile Gly Glu Ile Asp Glu Leu Asp Pro Lys Gly Val Lys Arg
                805                 810                 815
gta gtg atg tgt tct ggt aag gtt tat tac gac ctg ctg gaa cag cgt 2496
Val Val Met Cys Ser Gly Lys Val Tyr Tyr Asp Leu Leu Glu Gln Arg
            820                 825                 830
cgt aag aac aat caa cac gat gtc gcc att gtg cgt atc gag caa ctc 2544
Arg Lys Asn Asn Gln His Asp Val Ala Ile Val Arg Ile Glu Gln Leu
        835                 840                 845
tac ccg ttc ccg cat aaa gcg atg cag gaa gtg ttg cag cag ttt gct 2592
Tyr Pro Phe Pro His Lys Ala Met Gln Glu Val Leu Gln Gln Phe Ala
    850                 855                 860
cac gtc aag gat ttt gtc tgg tgc cag gaa gag ccg ctc aac cag ggc 2640
His Val Lys Asp Phe Val Trp Cys Gln Glu Glu Pro Leu Asn Gln Gly
865                 870                 875                 880
gca tgg tac tgc agc cag cat cat ttc cgt gaa gtg att ccg ttt ggg 2688
Ala Trp Tyr Cys Ser Gln His His Phe Arg Glu Val Ile Pro Phe Gly
                885                 890                 895
gct tct ctg cgt tat gca ggc cgc ccg gcc tcc gcc tct ccg gcg gta 2736
Ala Ser Leu Arg Tyr Ala Gly Arg Pro Ala Ser Ala Ser Pro Ala Val
            900                 905                 910
ggg tat atg tcc gtt cac cag aaa cag caa caa gat ctg gtt aat gac 2784
Gly Tyr Met Ser Val His Gln Lys Gln Gln Gln Asp Leu Val Asn Asp
        915                 920                 925
gcg ctg aac gtc gaa taa 2802
Ala Leu Asn Val Glu
    930

<210> 2
<211> 933
<212> PRT
<213> Escherichia coli

<400> 2
Met Gln Asn Ser Ala Leu Lys Ala Trp Leu Asp Ser Ser Tyr Leu Ser
1               5                   10                  15
Gly Ala Asn Gln Ser Trp Ile Glu Gln Leu Tyr Glu Asp Phe Leu Thr
            20                  25                  30
Asp Pro Asp Ser Val Asp Ala Asn Trp Arg Ser Thr Phe Gln Gln Leu
        35                  40                  45
Pro Gly Thr Gly Val Lys Pro Asp Gln Phe His Ser Gln Thr Arg Glu
    50                  55                  60
Tyr Phe Arg Arg Leu Ala Lys Asp Ala Ser Arg Tyr Ser Ser Thr Ile
65                  70                  75                  80
Ser Asp Pro Asp Thr Asn Val Lys Gln Val Lys Val Leu Gln Leu Ile
                85                  90                  95
Asn Ala Tyr Arg Phe Arg Gly His Gln His Ala Asn Leu Asp Pro Leu
            100                 105                 110
Gly Leu Trp Gln Gln Asp Lys Val Ala Asp Leu Asp Pro Ser Phe His

115                 120                 125
Asp Leu Thr Glu Ala Asp Phe Gln Glu Thr Phe Asn Val Gly Ser Phe
    130                 135                 140
Ala Ser Gly Lys Glu Thr Met Lys Leu Gly Glu Leu Leu Glu Ala Leu
145                 150                 155                 160
Lys Gln Thr Tyr Cys Gly Pro Ile Gly Ala Glu Tyr Met His Ile Thr
                165                 170                 175
Ser Thr Glu Glu Lys Arg Trp Ile Gln Gln Arg Ile Glu Ser Gly Arg
            180                 185                 190
Ala Thr Phe Asn Ser Glu Glu Lys Lys Arg Phe Leu Ser Glu Leu Thr
        195                 200                 205
Ala Ala Glu Gly Leu Glu Arg Tyr Leu Gly Ala Lys Phe Pro Gly Ala
    210                 215                 220
Lys Arg Phe Ser Leu Glu Gly Gly Asp Ala Leu Ile Pro Met Leu Lys
225                 230                 235                 240
Glu Met Ile Arg His Ala Gly Asn Ser Gly Thr Arg Glu Val Val Leu
                245                 250                 255
Gly Met Ala His Arg Gly Arg Leu Asn Val Leu Val Asn Val Leu Gly
            260                 265                 270
Lys Lys Pro Gln Asp Leu Phe Asp Glu Phe Ala Gly Lys His Lys Glu
        275                 280                 285
His Leu Gly Thr Gly Asp Val Lys Tyr His Met Gly Phe Ser Ser Asp
    290                 295                 300
Phe Gln Thr Asp Gly Gly Leu Val His Leu Ala Leu Ala Phe Asn Pro
305                 310                 315                 320
Ser His Leu Glu Ile Val Ser Pro Val Val Ile Gly Ser Val Arg Ala
                325                 330                 335
Arg Leu Asp Arg Leu Asp Glu Pro Ser Ser Asn Lys Val Leu Pro Ile
            340                 345                 350
Thr Ile His Gly Asp Ala Ala Val Thr Gly Gln Gly Val Val Gln Glu
        355                 360                 365
Thr Leu Asn Met Ser Lys Ala Arg Gly Tyr Glu Val Gly Gly Thr Val
    370                 375                 380
Arg Ile Val Ile Asn Asn Gln Val Gly Phe Thr Thr Ser Asn Pro Leu
385                 390                 395                 400
Asp Ala Arg Ser Thr Pro Tyr Cys Thr Asp Ile Gly Lys Met Val Gln
                405                 410                 415
Ala Pro Ile Phe His Val Asn Ala Asp Asp Pro Glu Ala Val Ala Phe
            420                 425                 430
Val Thr Arg Leu Ala Leu Asp Phe Arg Asn Thr Phe Lys Arg Asp Val
        435                 440                 445
Phe Ile Asp Leu Val Cys Tyr Arg Arg His Gly His Asn Glu Ala Asp
    450                 455                 460
Glu Pro Ser Ala Thr Gln Pro Leu Met Tyr Gln Lys Ile Lys Lys His
465                 470                 475                 480
Pro Thr Pro Arg Lys Ile Tyr Ala Asp Lys Leu Glu Gln Glu Lys Val
                485                 490                 495
Ala Thr Leu Glu Asp Ala Thr Glu Met Val Asn Leu Tyr Arg Asp Ala
            500                 505                 510
Leu Asp Ala Gly Asp Cys Val Val Ala Glu Trp Arg Pro Met Asn Met
        515                 520                 525
His Ser Phe Thr Trp Ser Pro Tyr Leu Asn His Glu Trp Asp Glu Glu
    530                 535                 540
Tyr Pro Asn Lys Val Glu Met Lys Arg Leu Gln Glu Leu Ala Lys Arg
545                 550                 555                 560
Ile Ser Thr Val Pro Glu Ala Val Glu Met Gln Ser Arg Val Ala Lys
                565                 570                 575
Ile Tyr Gly Asp Arg Gln Ala Met Ala Ala Gly Glu Lys Leu Phe Asp

580                 585                 590
Trp Gly Gly Ala Glu Asn Leu Ala Tyr Ala Thr Leu Val Asp Glu Gly
        595                 600                 605
Ile Pro Val Arg Leu Ser Gly Glu Asp Ser Gly Arg Gly Thr Phe Phe
    610                 615                 620
His Arg His Ala Val Ile His Asn Gln Ser Asn Gly Ser Thr Tyr Thr
625                 630                 635                 640
Pro Leu Gln His Ile His Asn Gly Gln Gly Ala Phe Arg Val Trp Asp
                645                 650                 655
Ser Val Leu Ser Glu Glu Ala Val Leu Ala Phe Glu Tyr Gly Tyr Ala
            660                 665                 670
Thr Ala Glu Pro Arg Thr Leu Thr Ile Trp Glu Ala Gln Phe Gly Asp
        675                 680                 685
Phe Ala Asn Gly Ala Gln Val Val Ile Asp Gln Phe Ile Ser Ser Gly
    690                 695                 700
Glu Gln Lys Trp Gly Arg Met Cys Gly Leu Val Met Leu Leu Pro His
705                 710                 715                 720
Gly Tyr Glu Gly Gln Gly Pro Glu His Ser Ser Ala Arg Leu Glu Arg
                725                 730                 735
Tyr Leu Gln Leu Cys Ala Glu Gln Asn Met Gln Val Cys Val Pro Ser
            740                 745                 750
Thr Pro Ala Gln Val Tyr His Met Leu Arg Arg Gln Ala Leu Arg Gly
        755                 760                 765
Met Arg Arg Pro Leu Val Val Met Ser Pro Lys Ser Leu Leu Arg His
    770                 775                 780
Pro Leu Ala Val Ser Ser Leu Glu Glu Leu Ala Asn Gly Thr Phe Leu
785                 790                 795                 800
Pro Ala Ile Gly Glu Ile Asp Glu Leu Asp Pro Lys Gly Val Lys Arg
                805                 810                 815
Val Val Met Cys Ser Gly Lys Val Tyr Tyr Asp Leu Leu Glu Gln Arg
            820                 825                 830
Arg Lys Asn Asn Gln His Asp Val Ala Ile Val Arg Ile Glu Gln Leu
        835                 840                 845
Tyr Pro Phe Pro His Lys Ala Met Gln Glu Val Leu Gln Gln Phe Ala
    850                 855                 860
His Val Lys Asp Phe Val Trp Cys Gln Glu Glu Pro Leu Asn Gln Gly
865                 870                 875                 880
Ala Trp Tyr Cys Ser Gln His His Phe Arg Glu Val Ile Pro Phe Gly
                885                 890                 895
Ala Ser Leu Arg Tyr Ala Gly Arg Pro Ala Ser Ala Ser Pro Ala Val
            900                 905                 910
Gly Tyr Met Ser Val His Gln Lys Gln Gln Gln Asp Leu Val Asn Asp
        915                 920                 925
Ala Leu Asn Val Glu
    930

<210>  3
<211>  927
<212>  DNA
<213>  Bacillus subtilis



<220>
<221>  CDS
<222>  (1)..(924)

<400>  3
atg agc cgt atc aga aaa gca ccc gct gga att tta ggt ttt ccg gtt          48

Met Ser Arg Ile Arg Lys Ala Pro Ala Gly Ile Leu Gly Phe Pro Val
1               5                   10                  15
gct cct ttt aac aca caa gga aaa ctg gag gaa gaa gct ctt ttt caa     96
Ala Pro Phe Asn Thr Gln Gly Lys Leu Glu Glu Glu Ala Leu Phe Gln
            20                  25                  30
aac att gag ttt ttg ctg aat gag ggc ttg gaa gcg ata ttt atc gct     144
Asn Ile Glu Phe Leu Leu Asn Glu Gly Leu Glu Ala Ile Phe Ile Ala
        35                  40                  45
tgc ggg tct ggt gaa ttc caa tct ctc agc cag aag gaa tat gag caa     192
Cys Gly Ser Gly Glu Phe Gln Ser Leu Ser Gln Lys Glu Tyr Glu Gln
    50                  55                  60
atg gtg gaa gtc gca gtc tcg gca gcc gga gga aag gtg ccg gtc tac     240
Met Val Glu Val Ala Val Ser Ala Ala Gly Gly Lys Val Pro Val Tyr
65                  70                  75                  80
acg ggg gtg ggc ggc aac ctc agc aca gcg ctt gat tgg gcg cag ctg     288
Thr Gly Val Gly Gly Asn Leu Ser Thr Ala Leu Asp Trp Ala Gln Leu
                85                  90                  95
tcg gag aaa aaa ggc gcc gac ggc tac ttg att ctg cct ccg tat ttg     336
Ser Glu Lys Lys Gly Ala Asp Gly Tyr Leu Ile Leu Pro Pro Tyr Leu
            100                 105                 110
gtg cat ggc gaa cag gag ggg ctg tac cag tat gcg aaa acg att att     384
Val His Gly Glu Gln Glu Gly Leu Tyr Gln Tyr Ala Lys Thr Ile Ile
        115                 120                 125
gaa agc act gac ttg aac gcg att ctt tat cag cgc gat aac gca gtt     432
Glu Ser Thr Asp Leu Asn Ala Ile Leu Tyr Gln Arg Asp Asn Ala Val
    130                 135                 140
ctc tct gtt gaa cag atc aaa cgt ctg acc gaa tgt gaa cag ctt gtc     480
Leu Ser Val Glu Gln Ile Lys Arg Leu Thr Glu Cys Glu Gln Leu Val
145                 150                 155                 160
ggt gtc aaa gac ggg gta gga aat atg gat ttg aat atc aat ctc gtc     528
Gly Val Lys Asp Gly Val Gly Asn Met Asp Leu Asn Ile Asn Leu Val
                165                 170                 175
tat acg atc ggc gac cgc ttg gga tgg ctc aac gga atg ccg atg gct     576
Tyr Thr Ile Gly Asp Arg Leu Gly Trp Leu Asn Gly Met Pro Met Ala
            180                 185                 190
gaa gtg acg atg ccg gct tat ctg ccg atc ggg ttc cac tct tac tct     624
Glu Val Thr Met Pro Ala Tyr Leu Pro Ile Gly Phe His Ser Tyr Ser
        195                 200                 205
tca gcg att tca aat tat atc ccg cat att tcg cga atg ttc tat gat     672
Ser Ala Ile Ser Asn Tyr Ile Pro His Ile Ser Arg Met Phe Tyr Asp
    210                 215                 220
gca ttg aaa aac gga aat gac gag ctt gtg aag gaa ctc tat cgg cat     720
Ala Leu Lys Asn Gly Asn Asp Glu Leu Val Lys Glu Leu Tyr Arg His
225                 230                 235                 240
gtg att ctg ccg atc aac gac att cgc aaa caa cga aaa ggc tac gcc     768
Val Ile Leu Pro Ile Asn Asp Ile Arg Lys Gln Arg Lys Gly Tyr Ala
                245                 250                 255
gta tcc ttg att aaa gcc ggt atg gaa atc atg ggt ttg aat gta agg     816
Val Ser Leu Ile Lys Ala Gly Met Glu Ile Met Gly Leu Asn Val Arg
            260                 265                 270
aat aca gcc aga ccg cct gtc ggc ccg gtt gaa aaa gat cat tat cag     864
Asn Thr Ala Arg Pro Pro Val Gly Pro Val Glu Lys Asp His Tyr Gln
        275                 280                 285
caa ttg gaa gca att ttg aag cag gcg gct gac cgt ttt ccg aaa aaa     912
Gln Leu Glu Ala Ile Leu Lys Gln Ala Ala Asp Arg Phe Pro Lys Lys
    290                 295                 300
gcc gcg acg gtt tga                                                 927
Ala Ala Thr Val

305

<210> 4
<211> 308
<212> PRT
<213> Bacillus subtilis

<400> 4
Met Ser Arg Ile Arg Lys Ala Pro Ala Gly Ile Leu Gly Phe Pro Val
1               5                   10                  15
Ala Pro Phe Asn Thr Gln Gly Lys Leu Glu Glu Glu Ala Leu Phe Gln
            20                  25                  30
Asn Ile Glu Phe Leu Leu Asn Glu Gly Leu Glu Ala Ile Phe Ile Ala
        35                  40                  45
Cys Gly Ser Gly Glu Phe Gln Ser Leu Ser Gln Lys Glu Tyr Glu Gln
    50                  55                  60
Met Val Glu Val Ala Val Ser Ala Ala Gly Gly Lys Val Pro Val Tyr
65                  70                  75                  80
Thr Gly Val Gly Gly Asn Leu Ser Thr Ala Leu Asp Trp Ala Gln Leu
                85                  90                  95
Ser Glu Lys Lys Gly Ala Asp Gly Tyr Leu Ile Leu Pro Pro Tyr Leu
            100                 105                 110
Val His Gly Glu Gln Glu Gly Leu Tyr Gln Tyr Ala Lys Thr Ile Ile
        115                 120                 125
Glu Ser Thr Asp Leu Asn Ala Ile Leu Tyr Gln Arg Asp Asn Ala Val
    130                 135                 140
Leu Ser Val Glu Gln Ile Lys Arg Leu Thr Glu Cys Glu Gln Leu Val
145                 150                 155                 160
Gly Val Lys Asp Gly Val Gly Asn Met Asp Leu Asn Ile Asn Leu Val
                165                 170                 175
Tyr Thr Ile Gly Asp Arg Leu Gly Trp Leu Asn Gly Met Pro Met Ala
            180                 185                 190
Glu Val Thr Met Pro Ala Tyr Leu Pro Ile Gly Phe His Ser Tyr Ser
        195                 200                 205
Ser Ala Ile Ser Asn Tyr Ile Pro His Ile Ser Arg Met Phe Tyr Asp
    210                 215                 220
Ala Leu Lys Asn Gly Asn Asp Glu Leu Val Lys Glu Leu Tyr Arg His
225                 230                 235                 240
Val Ile Leu Pro Ile Asn Asp Ile Arg Lys Gln Arg Lys Gly Tyr Ala
                245                 250                 255
Val Ser Leu Ile Lys Ala Gly Met Glu Ile Met Gly Leu Asn Val Arg
            260                 265                 270
Asn Thr Ala Arg Pro Pro Val Gly Pro Val Glu Lys Asp His Tyr Gln
        275                 280                 285
Gln Leu Glu Ala Ile Leu Lys Gln Ala Ala Asp Arg Phe Pro Lys Lys
    290                 295                 300
Ala Ala Thr Val
305

## Description

### TECHNICAL FIELD

The present invention relates to a synthetic pathway generating apparatus, a synthetic pathway generating method and a synthetic pathway generating program. The present invention also relates to a method of producing 3-hydroxypropionic acid, a method of producing crotonyl alcohol, and a method of producing biobutadiene.

### BACKGROUND ART

A bioprocess is broadly utilized as a method of producing industrially useful chemicals by making use of enzymatic reactions and metabolic reactions of microorganisms. Over the recent years, in addition to biogenically produced natural compounds, there arises a need for a new bioprocess for non-natural chemicals as products. A crucial key for developing the bioprocess for producing the non-natural chemicals is to find out novel bioreactions and to find out biosynthetic pathways by a rational method.

It is, however, difficult to rotationally predict unknown bioreactions that have been absolutely unknown, and the investigations made so far have been limited to those depending mainly on individualistic ideas. A novel bioreaction predicting method and a novel biosynthetic pathway design method, which are more rational but do not depend on the individualistic methods, are desired for developing the new bioprocesses.

A method for producing 3-hydroxypropionic acid is reported in Patent Document 1 filed by Cargill Inc. According to Patent Document 1, α-alanine is formed from pyruvate and then converted into β-alanine. The β-alanine is, in turn, converted via malonate semialdehyde into 3-hydroxypropionic acid.
As reported in Patent Document 2, a known method for producing biobutadiene comprises chemically converting butanol prepared by fermentation to synthesize butadiene. In such a process, it is required to convert butanol into butene via a dehydrating process and then to dehydrogenate butene. Thus, such a process is complex and consumes high energy, raising concerns over the increase in production costs.

### PRIOR ART DOCUMENTS

### PATENT DOCUMENTS

Patent document 1: International Publication Pamphlet No. WO 2006/022664
Patent document 2: International Publication Pamphlet No. WO 2010/099201

### NON PATENT DOCUMENTS

NON PATENT DOCUMENT 1: Ayoun Cho and four others, Prediction of novel synthetic pathways for the production of desired chemicals, BMC Systems Biology, 2010, 4:35. (doi:10.1186/1752-0509-4-35)
NON PATENT DOCUMENT 2: Christopher S. Henry and two others, Discovery and analysis of novel metabolic pathways for the biosynthesis of industrial chemicals: 3-hydroxypropanoate, Biotechnology and Bioengineering. (doi:10.1002/bit.22673)

### SUMMARY OF THE INVENTION

### PROBLEMS TO BE SOLVED BY THE INVENTION

Such an example is known that the same enzyme exhibits catalytic action on a plurality of substrates of which partial structures are coincident with each other. Therefore, a thinking way of predicting the new bioreaction has hitherto been employed based on the point that the partial structures of the substrates are coincident. A graph theory is a technique used for notating the structures of the compounds in a format recognizable to a computer and searching for the coincident partial structures between the compounds. In the case of implementing the search for the pathway by combining the graph theory with the biosynthetic pathway design method, a problem is that operations become complicated and calculations are highly time-consuming. Further, another bioreaction predicting method is that several types of chemical conversion rules in the enzymatic reactions are previously determined. This method is, however, deficient of scientific grounds to limit the chemical conversion rules to some rules, resulting in an apprehension that prediction accuracy might decline.

The biosynthetic pathway design method connotes a method enabling a pathway to a target compound to be searched for from a raw material compound in a way that exploits known information registered in a bioreaction information database and enabling a pathway to be selected, which has economic rationality and high feasibility in terms of a stoichiometric relationship and from a thermodynamic point of view, out of a plurality of candidate pathways. If exploiting only the known information registered in the bioreaction information database, however, the predicted pathways are, as a matter of course, confined to combinations of known reactions, and hence a new reaction pathway cannot be predicted. The present invention aims at generating a compound synthetic pathway without limiting chemical conversion rules.
The present invention aims at providing a novel method of producing 3-hydroxypropionic acid.
The present invention aims at providing novel methods of producing crotonyl alcohol and butadiene.

### MEANS FOR SOLVING THE PROBLEMS

The present invention adopts the following means in order to solve the problems. Namely, a synthetic pathway generating apparatus according to one aspect of the present invention includes: a converting unit to convert chemical structures of compounds into numerical values; a calculation unit to create a compound pair and to calculate a difference value by subtracting the numerical value of one compound of the compound pair from the numerical value of the other compound thereof; a determining unit to compare the difference value of the compound pair of the reaction between which is already known with the difference value of the compound pair of the reaction between which is unknown, and to determine the unknown reaction of the compound pair with the difference value being coincident as a virtual reaction; a generating unit to generate compound synthetic pathways by use of nodes representing the compounds and edges representing the known reaction and the virtual reaction respectively; and a selecting unit to select a synthetic pathway from a starting compound to a target compound out of the generated compound synthetic pathways. According to the synthetic pathway generating apparatus, a new compound synthetic pathway from the starting compound to the target compound can be selected out of the compound synthetic pathways generated by use of the nodes representing the compounds and edges representing the known reaction and the virtual reaction.

A synthetic pathway generating apparatus according to another aspect of the present invention includes: a converting unit to convert chemical structures of compounds into numerical values; a first calculation unit to create a compound pair and to calculate a first difference value by subtracting the numerical value of one compound of the compound pair from the numerical value of the other compound thereof; a second calculation unit to calculate a second difference value by subtracting the numerical value of a starting compound from the numerical value of a target compound; and a generating unit to generate, by use of the starting compound, the target compound and the plurality of first difference values, a synthetic pathway wherein a total value of the first difference values in the synthetic pathway from the starting compound to the target compound is coincident with the second difference value. According to the synthetic pathway generating apparatus, a new compound synthetic pathway from the starting compound to the target compound can be generated by generating the compound synthetic pathway configured from the starting compound, the target compound and the plurality of first difference values.

Further, the present invention may also be a computer, other devices, machines, etc. that execute any one of the processes described above. Still further, the present invention may also be a program for making the computer, other devices, machines, etc. realize any one of the functions described above. Yet further, the present invention may also be a recording medium recorded with such a program, which can be read by the computer etc.

The first aspect of the present invention relates to a method for producing 3-hydroxypropionic acid which comprises the step of reductively decarboxylating oxaloacetate to convert the oxaloacetate into 3-hydroxypropionic acid. The method for producing 3-hydroxypropionic acid may comprise culturing, in a medium containing a carbon source, a microorganism, which has an ability to produce 3-hydroxypropionic acid and an enzyme activity to reductively decarboxylate oxaloacetate to convert the oxaloacetate into 3-hydroxypropionic acid, thereby allowing to accumulate 3-hydroxypropionic acid in the medium or the cells; and collecting the 3-hydroxypropionic acid from the medium or the cells.

The second aspect of the present invention relates to a method for producing 3-hydroxypropionic acid which comprises the step of reductively decarboxylating oxaloacetate to convert the oxaloacetate into malonate; and the step of converting the malonate into 3-hydroxypropionic acid. The method for producing 3-hydroxypropionic acid may comprise culturing, in a medium containing a carbon source, a microorganism, which has an ability to produce 3-hydroxypropionic acid and the enzyme activity to reductively decarboxylate oxaloacetate to convert the oxaloacetate into malonate, thereby allowing to accumulate 3-hydroxypropionic acid in the medium or the cells; and collecting the 3-hydroxypropionic acid from the medium or the cells.

The third aspect of the present invention relates to a method for producing 3-hydroxypropionic acid which comprises the step of decarboxylating pyruvate to convert the pyruvate into formate; and the step of converting the formate into 3-hydroxypropionic acid. The method for producing 3-hydroxypropionic acid may comprise culturing, in a medium containing a carbon source, a microorganism, which has an ability to produce 3-hydroxypropionic acid and the enzyme activity to decarboxylate pyruvate to convert the pyruvate into formate, thereby allowing to accumulate 3-hydroxypropionic acid in the medium or the cells; and collecting the 3-hydroxypropionic acid from the medium or the cells.

The fourth aspect of the present invention relates to a method for producing 3-hydroxypropionic acid which comprises the step of converting C02107 ((S,S)-tartaric acid) into C00222 (3-oxopropanoate); and the step of converting the C00222 into 3-hydroxypropionic acid. The method for producing 3-hydroxypropionic acid may include culturing, in a medium containing a carbon source, a microorganism, which has an ability to produce 3-hydroxypropionic acid and the enzyme activity to convert C02107 into C00222, thereby allowing to accumulate 3-hydroxypropionic acid in the medium or the cells; and collecting the 3-hydroxypropionic acid from the medium or the cells.

The fifth aspect of the present invention relates to a method for producing crotonyl alcohol which comprises the step of converting acetyl-CoA into acetoacetyl-CoA; the step of converting the acetoacetyl-CoA into 3-hydroxybutyl-CoA; the step of converting the 3-hydroxybutyl-CoA into crotonyl-CoA; the step of converting the crotonyl-CoA into crotonyl aldehyde; and the step of converting the crotonyl aldehyde into crotonyl alcohol, and a method for producing butadiene further comprises the step of dehydrating the resultant crotonyl alcohol to give butadiene.
The method may comprise culturing, in a medium containing a carbon source, a microorganism, which has an ability to produce crotonyl alcohol, the enzyme activity to convert acetyl-CoA into acetoacetyl-CoA, the enzyme activity to convert acetoacetyl-CoA into 3-hydroxybutyl-CoA, the enzyme activity to convert 3-hydroxybutyl-CoA into crotonyl-CoA, the enzyme activity to convert crotonyl-CoA into crotonyl aldehyde, and the activity to convert crotonyl aldehyde into crotonyl alcohol, thereby allowing to accumulate crotonyl alcohol in the medium or the cells; and collecting the crotonyl alcohol from the medium or the cells, and may further comprises the step of dehydrating the resultant crotonyl alcohol to give butadiene.

### EFFECT OF THE INVENTION

According to the present invention, the compound synthetic pathway can be generated without limiting the chemical conversion rules.
According to the method of producing 3-hydroxypropionic acid of the present invention, 3-hydroxypropionic acid is efficiently produced.
According to the methods of producing crotonyl alcohol and butadiene of the present invention, crotonyl alcohol and butadiene are efficiently produced.

### BRIEF DESCRIPTION OF THE DRAWINGS

[FIG.1] A diagram of a configuration of a synthetic pathway generating apparatus 1 according to the present embodiment.
[FIG.2] A diagram illustrating an example in the case of grouping atom types with respect to compounds containing carbon (C) that are registered in a KEGG database.
[FIG.3] A diagram illustrating an example in the case of grouping the atom types with respect to compounds registered in a KEGG database.
[FIG.4] A diagram depicting an example in the case of counting the number of partial structures and the number of bonding states of the partial structures contained in a chemical structural formula of a compound (3-Hydroxypropionate).
[FIG.5] A diagram of a functional configuration of the synthetic pathway generating apparatus 1 according to the present embodiment.
[FIG.6] A flowchart illustrating a flow of generating the compound synthetic pathway based on a virtual reaction method.
[FIG.7] A diagram illustrating an example in the case of expressing a graph by graphic forms.
[FIG.8] A diagram illustrating information on the synthetic pathway.
[FIG.9] A flowchart illustrating a flow of generating the compound synthetic pathway based on an optimization method.
[FIG.10] A diagram illustrating an example of a compound synthetic pathway.
[FIG.11] A diagram illustrating an example of outputting information on the synthetic pathway.
[FIG.12] A diagram illustrating the compound synthetic pathway according to an example 2.
[FIG.13] A diagram illustrating the compound synthetic pathway according to an example 3.

### EMBODIMENTS FOR CARRYING OUT THE INVENTION

An embodiment of the present invention will hereinafter be described based on the drawings. A configuration of the following embodiment is an exemplification, and the present invention is not limited to the configuration of the embodiment.

### <Configuration of Synthetic Pathway Generating Apparatus 1>

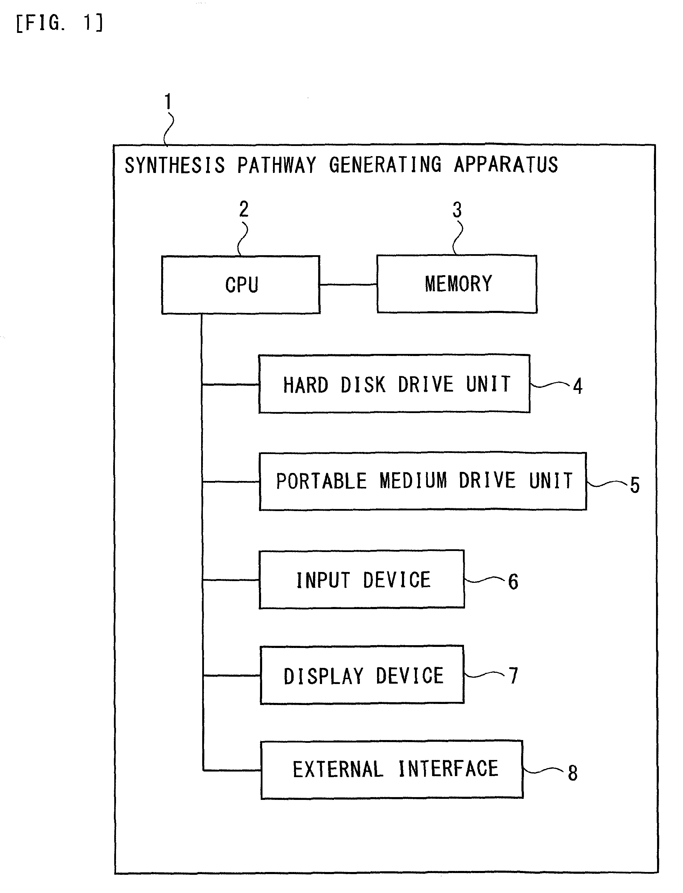

FIG. 1 is a diagram of a configuration of a synthetic pathway generating apparatus 1 according to the present embodiment. As depicted in FIG. 1, the synthetic pathway generating apparatus 1 includes a CPU (Central Processing Unit) 2, a memory 3, a hard disk drive unit 4, a portable medium drive unit 5, an input device 6, a display device 7 and an external interface 8.

The CPU 2 executes a computer program stored in the memory 3 and processes data stored in the memory 3, thereby controlling the synthetic pathway generating apparatus 1. The memory 3 gets stored with the program executed by the CPU 2 and the data processed by the CPU 2. The memory 3 includes a volatile RAM (Random Access Memory) and a nonvolatile ROM (Read Only Memory).

The hard disk drive unit 4 gets stored with the program loaded into the memory 3. Further, the hard disk drive unit 4 gets stored with the data processed by the CPU 2. The portable medium drive unit 5 is a drive unit for portable mediums such as a CD (Compact Disc), a DVD (Digital Versatile Disk), a HD-DVD (High Definition-DVD) and a Blu-ray disc. Moreover, the portable medium drive unit 5 may also be an input/output device for a card medium including a nonvolatile memory such as a flash memory. The portable medium driven by the portable medium drive unit 5 retains, e.g., the computer program installed into the hard disk drive unit 4, the input data, etc. The input device 6 is exemplified by, e.g., a keyboard, a mouse, a pointing device, a wireless remote controller, and so on.

The display device 7 displays the data processed by the CPU 2 and the data stored in the memory 3. The display device 7 is exemplified such as a liquid crystal display device, a plasma display panel, a CRT (Cathode Ray Tube) and an electroluminescence panel.

The external interface 8 connects the synthetic pathway generating apparatus 1 to an external network. The external interface 8 is equipped with communication devices, e.g., a modem, a terminal adaptor, etc. and controls communications between the synthetic pathway generating apparatus 1 and equipment on the external network. The external network is exemplified by, e.g., the Internet and a local area network (LAN). Further, the external network may be built up by communication lines such as a telephone line, a leased line, an optical communication network and a communication satellite.

The synthetic pathway generating apparatus 1 according to the present embodiment generates a compound synthetic pathway from a starting compound to a target compound. There are two types of methods of generating the compound synthetic pathway, which are a virtual reaction method and an optimization method. The virtual reaction method and the optimization method provide definitions of the compounds by their unique expression methods.

### <One Example of Procedures for Defining Compounds by Unique Expression Methods>

To start with, one example of procedures for defining the compounds by a unique expression method will be described. The procedures involve classifying partial structures contained a chemical structural formula of the compound according to predetermined standards, and defining the partial structures contained the chemical structural formula of the compound as character strings. The predetermined standards may be standards by which the structures of the compounds are strictly defined and may also be standards taking similarities of the structures of the compounds into consideration. Further, the predetermined standards may also be standards based on distinctions among functional groups (alkane, alkene, etc.). For example, the partial structures contained the chemical structural formula of the compound may be classified according to atom types registered in a database of KEGG (Kyoto Encyclopedia of Genes and Genomes). Moreover, the atom types registered in the database of KEGG are grouped, and the partial structures contained the chemical structural formula of the compound may also be classified according to these grouped atom types.

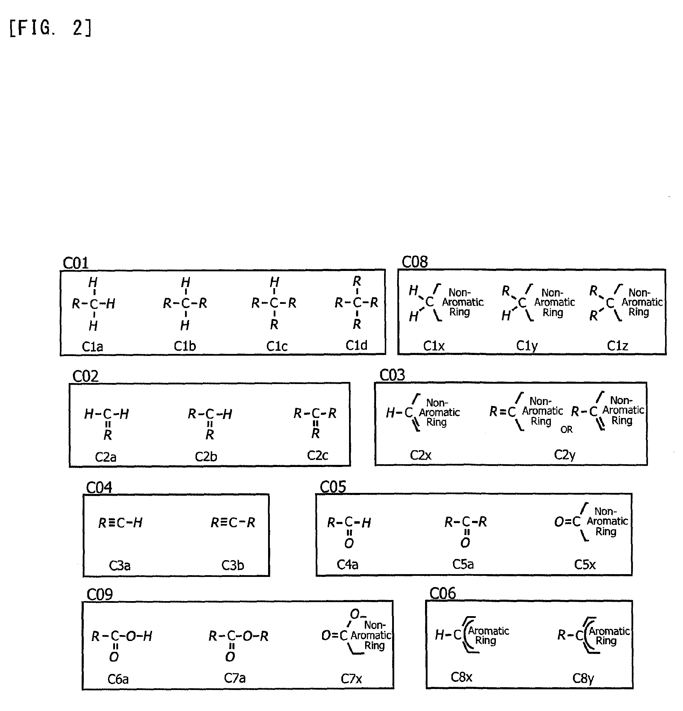

FIG. 2 depicts an example of a case of grouping the atom types with respect to the compounds containing carbon (C), which are registered in the KEGG database. The atom types, though grouped based on similarities of chemical reactions in FIG. 2, may also be grouped based on the distinctions among the functional groups (alkane, alkene, etc.). In FIG. 2, C1a, C1b, C1c and C1d are identification numbers allocated to the atom types registered in the KEGG database. The same is applied to C2a, C3a, etc. in FIG. 2. In FIG. 2, C01 is an identification number allocated to data (information) organized when grouping C1a, C1b, C1c and C1d. The same is applied to C02, C03 and so on in FIG. 2.

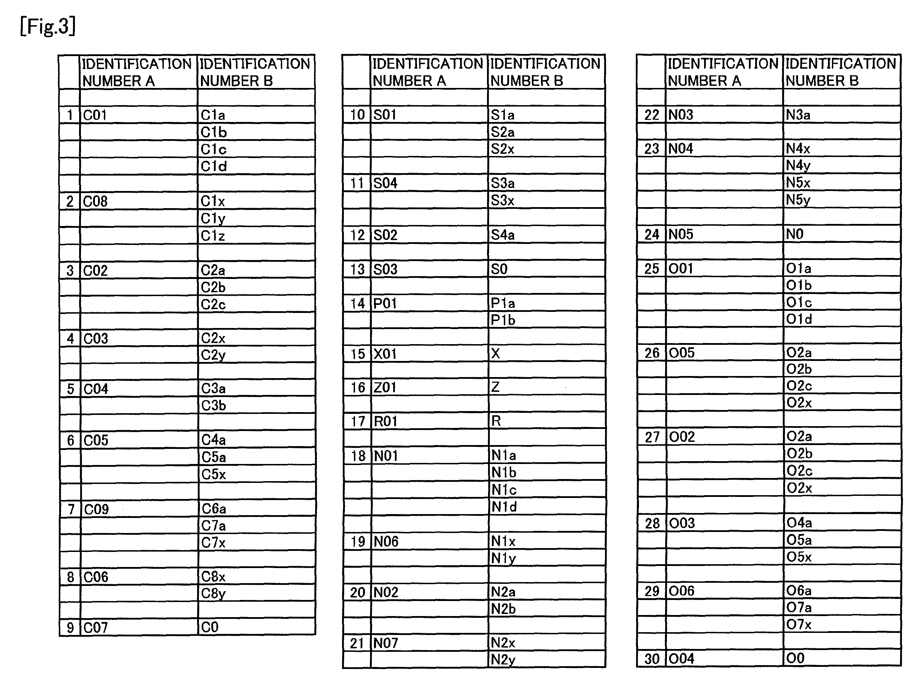

FIG. 3 is an example in the case of grouping the atom types about the compounds registered in the KEGG database. Identification numbers allocated to the atom types about the compounds registered in the KEGG database are inputted to an "identification number A" field in FIG. 3. Identification numbers allocated to pieces of data organized when grouping the atom types about the compounds registered in the KEGG database are inputted to an "identification number B" field in FIG. 3.

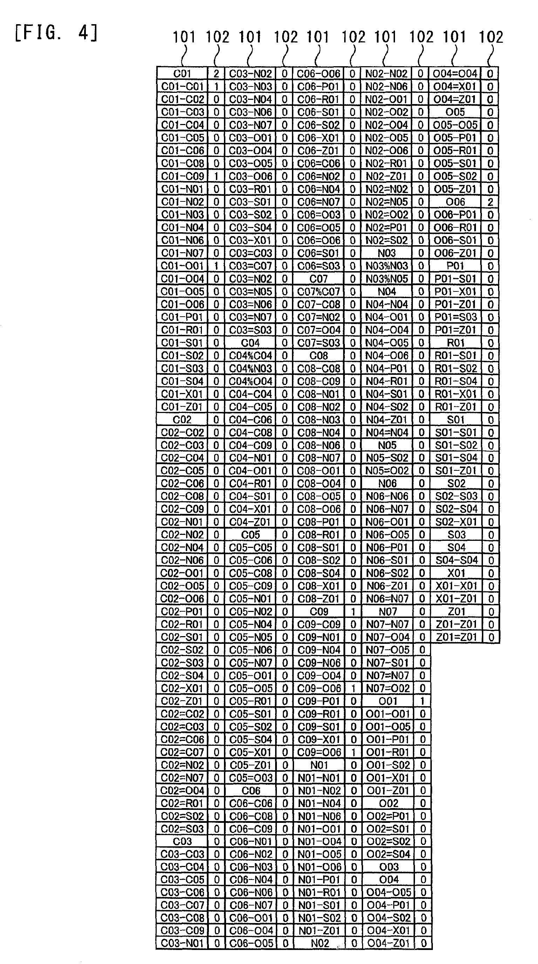

FIG. 4 is an example in the case of counting the number of the partial structures and the number of bonding states of the partial structures contained in a chemical structural formula of a compound (3-Hydroxypropionate). In FIG. 4, the number of the partial structures contained in the chemical structural formula of the compound (3-Hydroxypropionate) is counted per type of the partial structure, and the number of the bonding states of the partial structures contained in the chemical structural formula of the compound (3-Hydroxypropionate) is counted per type of the partial structure and per type of the bonding state.

The identification numbers allocated to the data organized when grouping the atom types with respect to the compounds registered in the KEGG database are inputted to a field specified by the numeral "101" in FIG. 4. Numerical values given after counting the number of the partial structures contained in the chemical structural formulae of the compounds on the per-type basis of the partial structure are inputted to a field specified by the numeral "102" in FIG. 4. Further, numerical values given after counting the number of the bonding states of the partial structures contained in the chemical structural formulae of the compounds on the per-type basis of the partial structure and on the per-type basis of the bonding state, are inputted to the field specified by the numeral "102" in FIG. 4. Note that single bond is notated by "-", double bond is notated by "=", and triple bond is notated by "%" in FIG. 4. In the present embodiment, what consecutively arranges the numerical values inputted to the field specified by the numeral "102" in FIG. 4 is described by UF (Universal Feature). As depicted in FIG. 4, the UF of the compound is assembled from atomic information representing the number of the partial structures contained in the chemical structural formulae of the compounds and from bonding information representing the number of the bonding states of the partial structures contained in the chemical structural formulae of the compounds.

### <Functional Configuration of Synthetic Pathway Generating Apparatus 1>

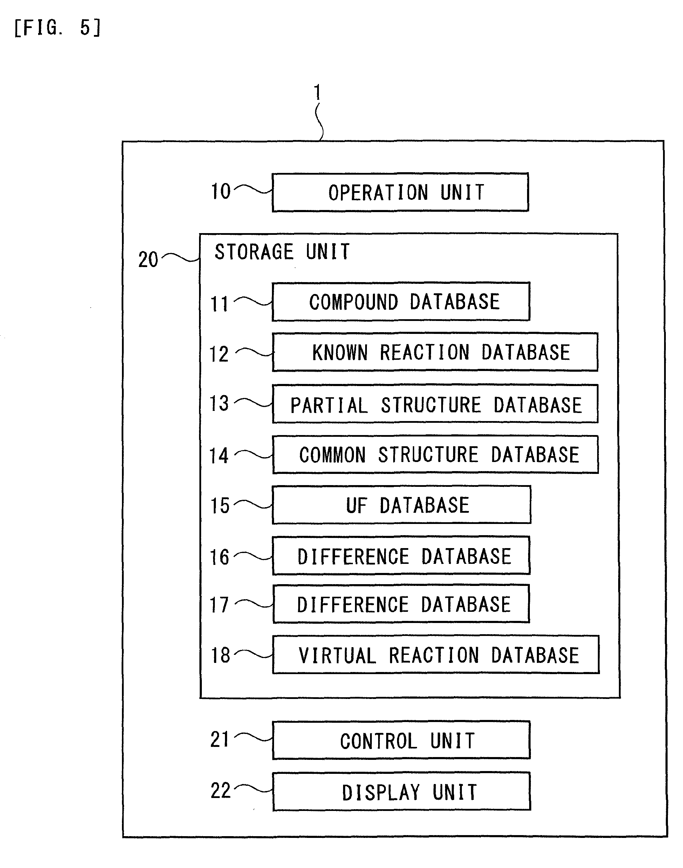

FIG. 5 is a diagram of a functional configuration of the synthetic pathway generating apparatus 1 according to the present embodiment. The synthetic pathway generating apparatus 1 illustrated in FIG. 5 includes an operation unit 10 that operates the synthetic pathway generating apparatus 1 upon accepting a user's operation, a storage unit 20 incorporating a compound database 11, a known reaction database 12, a partial structure database 13, a common structure database 14, a UF database 15, difference databases 16, 17 and a virtual reaction database 18, a control unit 21 that executes a variety of processes, and a display unit 22 that displays various items of information. These respective function units are realized by the CPU 2, the memory 3, etc. and computer programs executed by the CPU 2.

### <Compound Database 11>

The compound database 11 is stored with nomenclatures of the compounds and the data of the chemical structural formulae of the compounds in the way of being associated with each other. For example, the nomenclatures of the compounds and the data of the chemical structural formulae of the compounds, which are registered in the KEGG database, may also be stored in the compound database 11 in the way of being associated with each other. Further, in addition to the compounds registered in the KEGG database, the compound database 11 may also be stored with the compound nomenclatures of the target compounds (including non-natural compounds) and the data of the chemical structural formulae of the compounds in the way of being associated with each other. Moreover, e.g., the compound database 11 may be stored with the nomenclatures of the compounds registered in a metabolite database such as the PubChem database, the BRENDA database and the MetaCyc database and with the data of the chemical structural formulae of the compounds in the way of being associated with each other. Without being limited to this storage, the compound database 11 may also be stored with the nomenclatures of the compounds and the data of the chemical structural formulae of the compounds, which are registered in a variety of chemical substance databases, in the way of being associated with each other.

In the present embodiment, with respect to the compounds stored in the compound database 11, a reaction in such a compound pair that a compound (e.g., a compound B) is, it is known, synthesized from a compound (e.g., a compound A) is referred to as a known reaction. Information about whether to be the known reaction or not can be set by a user, however, for instance, the reactions registered in the KEGG database may be set as the known reactions.

In the present embodiment, with respect to the compounds stored in the compound database 11, such a compound pair that the compound (e.g., the compound B) is, it is known, synthesized from the compound (e.g., the compound A) is referred to as a known compound pair. Here at, a compound A' defined as an intermediary body may exist between the compound A and the compound B.

In the present embodiment, in regard to the compounds stored in the compound database 11, a reaction in such a compound pair that a certain compound (e.g., a compound D) is, it is not known, synthesized from a certain compound (e.g., a compound C) is referred to as an unknown reaction. In the present embodiment, as for the compounds stored in the compound database 11, such a compound pair that a certain compound (e.g., the compound D) is, it is not known, synthesized from a certain compound (e.g., the compound C) is referred to as an unknown compound pair. In the present embodiment, in connection with the compounds stored in the compound database 11, an enzyme used for the reaction in such a compound pair that the compound (e.g., the compound B) is, it is known, synthesized from the compound (e.g., the compound A) is referred to as a known reaction enzyme.

### <Known Reaction Database 12>

The known reaction database 12 is stored with the nomenclatures of the respective compounds in the known compound pairs, reaction directions in the known compound pairs and the known reaction enzymes in the way of being associated with each other. There may be a plurality of known reaction enzymes.

For example, the nomenclatures of the respective compounds in the compound pairs registered in the KEGG database, the reaction directions of the reactions of the compound pairs and the enzymes used for the reactions in the compound pairs may be stored in the known reaction database 12 in the way of being associated with each other. Further, the known reaction database 12 may be stored with, e.g., the nomenclatures of the respective compounds in the compound pairs, the reaction directions in the compound pairs and the enzymes used for the reactions in the compound pairs, which are registered in the metabolite database such as the PubChem database, the BRENDA database and the MetaCyc database in the way of being associated with each other. Furthermore, the known reaction database 12 may also be stored with the nomenclatures of the respective compounds in the compound pairs, the reaction directions in the compound pairs and the enzymes used for the reactions in the compound pairs, which are registered in a metabolic reaction database of *Escherichia coli* in the way of being associated with each other. Without being limited to this storage, the known reaction database 12 may also be stored with the nomenclatures of the respective compounds in the compound pairs, the reaction directions in the compound pairs and the enzymes used for the reactions in the compound pairs, which are registered in the variety of chemical substance databases, in the way of being associated with each other.

### <Partial Structure Database 13>

The partial structure database 13 is stored with items of partial data of the chemical structural formulae of the compounds, and identification numbers allocated to the respective items of partial data of the chemical structural formulae of the compounds. The partial data of the chemical structural formulae of the compounds are data created by extracting partial chemical structures from the chemical structural formulae of the compounds according to the predetermined standards. For instance, the partial data of the chemical structural formulae of the compounds may also be created by extracting the partial chemical structures from the chemical structural formulae of the compounds registered in the compound database 11 on the basis of the atom types registered in the KEGG database. In the present embodiment, the identification number allocated to the partial data of the chemical structural formula of the compound is referred to as a structure number. Take FIG. 2 for example, C1a, C1b, C1c and C1d correspond to the structure numbers.

### <Common Structure Database 14>

The common structure database 14 is stored with data given when grouping the partial data of the chemical structural formulae of the compounds and identification numbers allocated to the data given when grouping the partial data. In the present embodiment, the identification numbers allocated to the data given when grouping the partial data of the chemical structural formulae of the compounds are referred to as common structure numbers. For instance, to give an example of FIG. 2, C01 corresponds to the common structure number.

### <UF Database 15>

The UF database 15 is stored with the UFs of the compounds in the way of being associated with the nomenclatures of the compounds.

### <Creation of UF of Compound>

The control unit 21 creates UFs of the compounds stored in the compound database 11. Then, the control unit 21 stores the created UFs of the compounds in the UF database 15 in a way that associates the UFs of the created compounds with the nomenclatures of the compounds.

Note that with respect to the UFs of the compounds and the nomenclatures of the compounds which are stored in the UF database 15, there exists a case where some compounds have the same UFs but are different in their nomenclatures. For example, an assumption is that the compound A and the compound B are classified by use of the example in FIG. 2, in which the compound A contains two pieces of "C1a", and the compound B contains one piece of "C1a" and one piece of "C1b". Both of the compound A and the compound B have two pieces of "C01" because of C1a and C1b being grouped, on which assumption the UFs of the compounds are created. For this reason, there is the case in which the UFs of the compounds are the same, but the nomenclatures of the compounds are different. In this case, the UF database 15 is stored with the nomenclatures of the plurality of compounds in the way of being associated with the UF of the compound.

In the description given above, the control unit 21 creates the UFs of the compounds by referring to the common structure database 14. To be specific, the control unit 21 creates the UFs of the compounds by referring to the data given when grouping the partial data of the chemical structural formulae of the compounds and the common structure numbers. In the present embodiment, however, without being limited to this reference mode, the control unit 21 may create the UFs of the compounds by referring to the partial structure database 13. Namely, the control unit 21 may create the UFs of the compounds by referring to the partial data of the chemical structural formulae of the compounds and the structure numbers thereof.

### <Difference Database 16>

The difference database 16 is stored with differences between the UFs of the compound pairs and with the nomenclatures of the respective compounds of the compound pairs in the way of being associated with each other. For instance, a value obtained by subtracting the UF of the compound B from the UF of the compound A is defined as a difference between the UFs of the compound pairs. Note that the difference database 16 may be stored with the UFs of the respective compounds of the compound pairs in place of the nomenclatures of the respective compounds of the compound pairs.

### <Creation of DF Stored in Difference Database 16>

The control unit 21 creates pairs of overall compounds stored in the UF database 15, and calculates the differences between the UFs of the created compound pairs. In the present embodiment, the difference between the UFs of the compound pairs is referred to as a DF (Difference Feature).

The control unit 21 creates, based on the presumption that the reaction occurs in the overall compound pairs stored in the UF database 15, the pairs of overall compound pairs stored in the UF database 15. For example, the compounds A, B and C are stored in the UF database 15, in which case the control unit 21 creates the pair of the compound A and the compound B, the pair of the compound A and the compound C and the pair of the compound B and the compound C. Furthermore, in the case of calculating the DF of the pair of the compound A and the compound B, the control unit 21 calculates a value by subtracting the UF of the compound B from the UF of the compound A and a value by subtracting the UF of the compound A from the UF of the compound B. Then, the control unit 21 stores the DFs and the nomenclatures of the respective compounds of the compound pairs in the difference database 16 in the way of being associated with each other.

### <Difference Database 17>

The difference database 17 is stored with the differences between the UFs of the known compound pairs, the nomenclatures of the respective compounds of the known compound pairs and the known reaction enzymes in the way of being associated with each other. Note that the difference database 17 may also be stored with the UFs of the respective compounds of the known compound pairs in place of the nomenclatures of the respective compounds of the known compound pairs.

### <Creation of DF Stored in Difference Database 17>

The control unit 21 calculates the difference between the UFs of the known compound pairs in the compounds stored in the UF database 15. In the present embodiment, the difference between the UFs of the known compound pairs is referred to as a known DF. For instance, the reaction of synthesizing the compound B from the compound A is a known reaction, in which case the control unit 21 calculates the known DF by subtracting the UF of the compound A from the UF of the compound B. Then, the control unit 21 stores the known DFs, the nomenclatures of the respective compounds of the known compound pairs and the known reaction enzymes in the difference database 17 in the way of being associated with each other. The control unit 21 uses the data stored in the known reaction database 12 for the nomenclatures of the respective compounds of the known compound pairs and for the known reaction enzymes.

### <Generation of Compound Synthetic Pathway by Virtual Reaction Method>

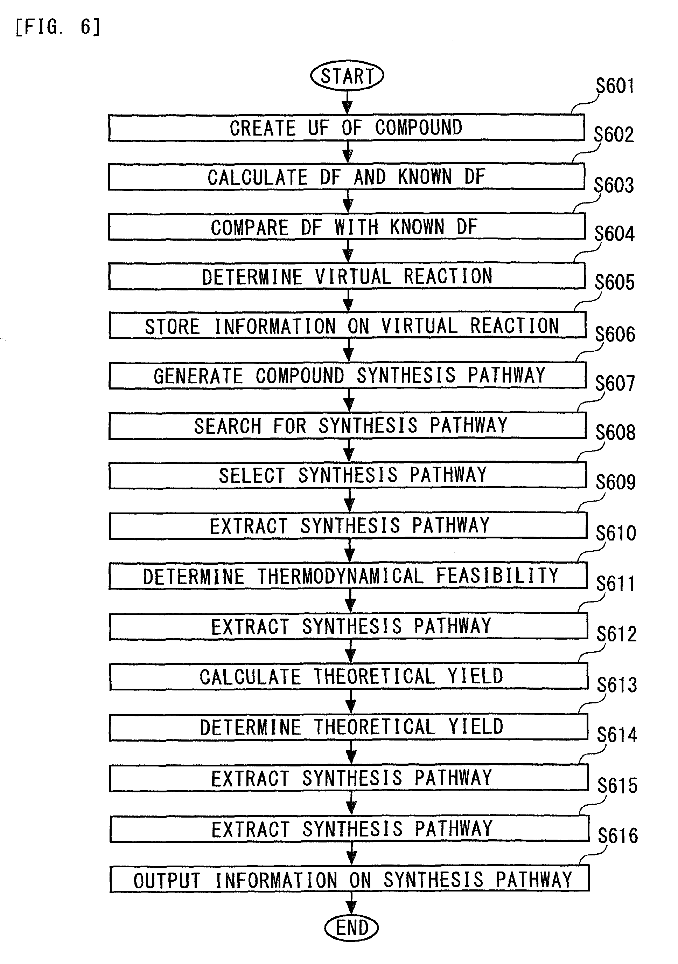

A flow of generating the compound synthetic pathway by the virtual reaction method will be described with reference to FIG. 6. A start of the flow in FIG. 6 is triggered by accepting an instruction to generate the compound synthetic pathway by the virtual reaction method from a user via the operation unit 10. On this occasion, designations of the starting compound and the target compound may also be accepted from the user via the operation unit 10.

In a process in S601 of FIG. 6, the control unit 21 creates the UFs of the compounds stored in the compound database 11. To be specific, the control unit 21 counts the number of the partial structures contained in the chemical structural formulae of the compounds stored in the compound database 11 on the per-type basis of the partial structure, and counts the number of the bonding states of the partial structures contained in the chemical structural formulae of the compounds on the per-type basis of the partial structure and on the per-type basis of the bonding state, thereby converting the chemical structural formulae of the compounds into numerical values. Further, in the process in S601 of FIG. 6, the control unit 21 stores the created UFs and the nomenclatures of the compounds in the UF database 15 in the way of being associated with each other. The control unit 21 executing the process in S601 of FIG. 6 functions as a converting unit.

Next, in a process of S602 in FIG. 6, the control unit 21 creates the pairs of overall compounds stored in the UF database 15, and calculates the DFs of the created compound pairs. The control unit 21 stores the calculated DFs and the nomenclatures of the respective compounds of the compound pairs in the difference database 16 in the way of being associated with each other.

Moreover, in the process of S602 in FIG. 6, the control unit 21 calculates the known DFs of the known compound pairs in the compound pairs stored in the UF database 15. The control unit 21 stores the thus calculated known DFs, the nomenclatures of the respective compounds of the known compound pairs and the known reaction enzymes in the difference database 17 in the way of being associated with each other. The control unit 21 executing the process of S602 in FIG. 6 functions as a calculation unit.

Then, in a process of S603 in FIG. 6, the control unit 21 compares the DFs stored in the difference database 16 with the known DFs stored in the difference database 17.

Subsequently, in a process of S604 in FIG. 6, the control unit 21 determines, as a virtual reaction, an unknown reaction in an unknown compound pair with respect to the DF coincident with the known DF stored in the difference database 17 in the DFs stored in the difference database 16. It is predicted that the unknown reaction will occur in the unknown compound pair with respect to the DF coincident with the known DF. It is to be noted that the control unit 21 does not determine, as the virtual reaction, the known reaction in the known compound pair with respect to the DF coincident with the known DF stored in the difference database 17 in the DFs stored in the difference database 16.

Further, in the process of S604 in FIG. 6, the control unit 21, if the DF stored in the difference database 16 is coincident with the known DF stored in the difference database 17, determines the known reaction enzyme associated with the coincident known DF as the enzyme used for the virtual reaction. The control unit 21, if there is a plurality of known reaction enzymes associated with the known DF, determines the plurality of known reaction enzymes as the enzymes used for the virtual reaction. The control unit 21 executing the processes of S603 and S604 in FIG. 6 functions as a determining unit.

Next, in the process of S605 in FIG. 6, the control unit 21 stores the nomenclatures of the respective compounds of the compound pairs related to the virtual reactions and the enzymes used for the virtual reactions in the virtual reaction database 18 in the way of being associated with each other.

Then, in the process of S606 in FIG. 6, the control unit 21 generates the compound synthetic pathway on the basis of the known reaction database 12 and the virtual reaction database 18. In this case, the control unit 21 generates the compound synthetic pathway by use of nodes (apexes) indicating the respective compounds of the known compound pairs and the respective compounds of the compound pairs related to the virtual reaction and by use of edges (sides) indicating the known reactions and the virtual reactions. Namely, the control unit 21 configures a graph (network) including the overall reactions, in which the nodes represent the respective compounds of the known compound pairs and the respective compounds of the compound pairs related to the virtual reactions, and the edges represent the known reactions and the virtual reactions, thus generating the compound synthetic pathway. The control unit 21 refers to the known reaction database 12 about the respective compounds of the known compound pairs and the known reactions, and refers to the virtual reaction database 18 about the respective compounds of the compound pairs related to the virtual reactions and about the virtual reactions. The control unit 21 executing the process of S606 in FIG. 6 functions as a generating unit.

The graph is formally defined as an ordered pair *G* = (*V*, *E*) including a set V of the nodes and a set E of the edges that connect the nodes to each other. The ordered pair G serves to express how the compounds contained in the graph are adjoined (how the network is configured) via the respective reactions. The graph may be expressed by graphic forms and may also be expressed by an array processing in which respective elements of arrays serve as the nodes.

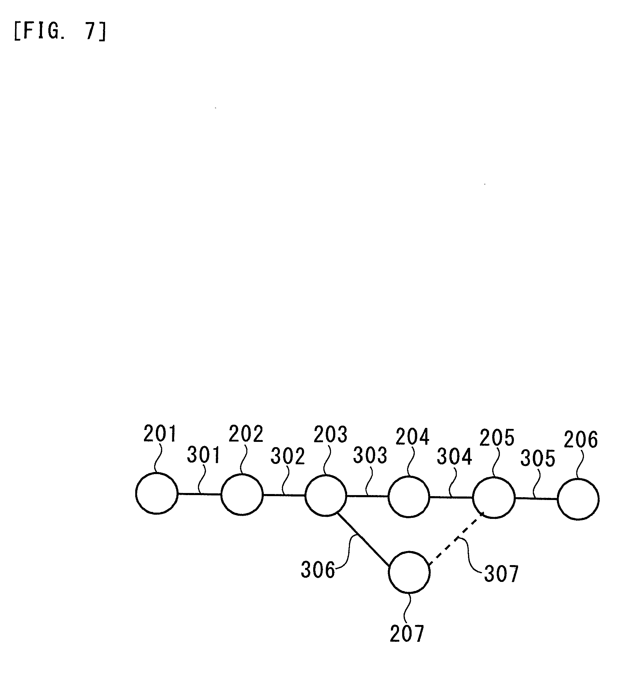

FIG. 7 illustrates an example in the case of expressing the graph by using the graphic forms. In FIG. 7, the respective compounds of the known compound pairs and the respective compounds of the compound pairs related to the virtual reactions are indicated by nodes 201 - 207, while the known reactions and the virtual reactions are indicated by edges 301 - 307. Further, in FIG. 7, the edge depicted by a solid line represents the known reaction, while the edge depicted by a broken line represents the virtual reaction.

Subsequently, in a process of S607 in FIG. 6, the control unit 21 searches for the synthetic pathway from the starting compound to the target compound out of the compound synthetic pathways generated in the process of S606 in FIG. 6. The control unit 21 may search for the synthetic pathway from the starting compound to the target compound, which involves a predetermined or less number of reaction steps, out of the compound synthetic pathways generated in the process of S606 in FIG. 6. The control unit 21 may search for the synthetic pathway from the starting compound to the target compound by a depth-first search or a breadth--first search, etc. out of the compound synthetic pathways generated in the process of S606 in FIG. 6. The control unit 21 may implement a method (forward search algorithm) in which the starting point is the starting compound or a raw material compound, while the ending point is the target compound, or a method (backward search algorithm) in which the starting point is the target compound, while the ending point is the starting compound or the raw material compound. The raw material compound is the compound becoming a raw material in the case of synthesizing the target compound. It may be determined which method, the forward search algorithm or the backward search algorithm, is implemented according to a purpose of the pathway search. The raw material compound may be designated by accepting the designation from the user via the operation unit 10.

Next, in a process of S608 in FIG. 6, the control unit 21 selects the synthetic pathway from the starting compound to the target compound out of the compound synthetic pathways generated in the process of S606 in FIG. 6 (procedure 1). In the process of S608 in FIG. 6, as a substitute for the procedure 1, the control unit 21 may select the synthetic pathway from the starting compound to the target compound, which involves the predetermined or less number of reaction steps, out of the compound synthetic pathways generated in the process of S606 in FIG. 6 (procedure 2). In the process of S608 in FIG. 6, in place of the procedure 1, the control unit 21 may select the synthetic pathway from the starting compound to the target compound, which involves the predetermined number of reaction steps or a predetermined number of reaction step ranges, out of the compound synthetic pathways generated in the process of S606 in FIG. 6 (procedure 3). The control unit 21 executing the process of S608 in FIG. 6 functions as a selecting unit.

For example, in FIG. 7, the node 201 indicates the raw material compound, the node 203 indicates the starting compound, and the node 205 indicates the target compound, in which case a synthetic pathway configured by including the nodes 203, 204, 205 and the edges 303, 304 and a synthetic pathway configured by including the nodes 203, 207, 205 and the edges 306, 307 are selected as the synthetic pathway from the starting compound to the target compound. The nodes 204 and 207 are intermediary compounds.

Then, in a process of S609 in FIG. 6, the control unit 21 extracts the synthetic pathway from the starting compound to the target compound, which involves a predetermined or less number of virtual reaction steps, out of the compound synthetic pathways from the starting compound to the target compound that are selected in the process of S608 in FIG. 6 (procedure A). In the process of S609 in FIG. 6, as a substitute for the procedure A, the control unit 21 may extract the synthetic pathway from the starting compound to the target compound, which involves the predetermined number of virtual reaction steps or a predetermined number of virtual reaction step ranges, out of the compound synthetic pathways from the starting compound to the target compound that are selected in the process of S608 in FIG. 6 (procedure B).

Subsequently, in a process of S610 in FIG. 6, the control unit 21 makes a determination as to thermodynamic feasibility about the synthetic pathway from the starting compound to the target compound, which is extracted in the process of S609 in FIG. 6.

The determination as to the thermodynamic feasibility is made by calculating a change of free energy in each reaction contained in the synthetic pathway from the starting compound to the target compound that is extracted in the process of S609 in FIG. 6 by a method described in, e.g., Biophys. J. Vol.92, No.7, pp1792-1805 (2007) and by determining a direction of each reaction on the basis of a calculation result. If the respective reactions contained in the synthetic pathway from the starting compound to the target compound that is extracted in the process of S609 in FIG. 6 are a forward reaction and a reversible reaction, the control unit 21 determines that there is the thermodynamic feasibility with respect to the synthetic pathway from the starting compound to the target compound that is extracted in the process of S609 in FIG. 6. Moreover, if a reverse reaction exists in the respective reactions contained in the synthetic pathway from the starting compound to the target compound that is extracted in the process of S609 in FIG. 6, the control unit 21 determines that there is none of the thermodynamic feasibility with respect to the synthetic pathway from the starting compound to the target compound that is extracted in the process of S609 in FIG. 6.

Next, in a process of S611 in FIG. 6, the control unit 21 extracts the synthetic pathway exhibiting the thermodynamic feasibility out of the compound synthetic pathways from the starting compound to the target compound that are extracted in the process of S609 in FIG. 6. The control unit 21 executing the processes of S610 and S611 in FIG. 6 functions as a thermodynamic determination unit.

Then, in a process of S612 in FIG. 6, the control unit 21 calculates a (molar) theoretical yield (%) of the target compound synthesized by the synthetic pathway extracted in the process of S611 in FIG. 6. In this case, the control unit 21 may also calculate the (molar) theoretical yield (%), derived from the raw material compound, of the target compound synthesized by the synthetic pathway extracted in the process of S611 in FIG. 6. The control unit 21, in the case of calculating the (molar) theoretical yield (%), derived from the raw material compound, of the target compound synthesized by the synthetic pathway extracted in the process of S611 in FIG. 6, refers to the known reaction database 12 and thus generates the synthetic pathway from the raw material compound to the starting compound. Then, the control unit 21 calculates the (molar) theoretical yield (%), derived from the raw material compound, of the target compound synthesized by the synthetic pathway extracted in the process of S611 in FIG. 6 by use of the synthetic pathway from the raw material compound to the starting compound and the synthetic pathway extracted in the process of S611 in FIG. 6. The raw material compound may be designated by accepting the designation from the user via the operation unit 10. In this case, the control unit 21 may generate the synthetic pathway from the raw material compound to the starting compound by referring to the data pertaining to the metabolic reaction of the *Escherichia coli*, which are stored in the known reaction database 12. The calculation of the (molar) theoretical yield can be carried out by a method described in, e.g., Biotechnology and Bioengineering, Volume 42, p.59-73 (1993).

Subsequently, in a process of S613 in FIG. 6, the control unit 21 determines whether the (molar) theoretical yield (%) calculated in the process of S612 in FIG. 6 is equal to or larger than a threshold value or not (alternatively whether larger than the threshold value or not). The threshold value can be arbitrarily set to an arbitrary value such as 0%, 100% and 200%.

Next, in a process of S614 in FIG. 6, the control unit 21 extracts the synthetic pathway for synthesizing the target compound of which the (molar) theoretical yield (%) calculated in the process of S612 in FIG. 6 is equal to or larger than the threshold value (alternatively the (molar) theoretical yield (%) is larger than the threshold value) out of the compound synthetic pathways extracted in the process of S611 in FIG. 6. The control unit 21 executing the processes of S612, S613 and S614 in FIG. 6 functions as a (molar) theoretical yield determining unit.

Then, in a process of S615 in FIG. 6, the control unit 21 extracts a practical synthetic pathway from the synthetic pathway extracted in the process of S614 in FIG. 6. The practical synthetic pathway may be extracted in a way that evaluates a change in carbon number of each reaction that is contained in the synthetic pathway and a magnitude of the carbon number and takes account of a degree of difficulty of altering the individual virtual reaction enzymes.

Further, the control unit 21 may previously limit the virtual reaction on the synthetic pathway from the starting compound to the target compound that is selected in the process of S608 in FIG. 6 to the enzymatic reaction with a past result of the alteration by referring to information etc. obtained in advance. Moreover, the control unit 21 may narrow down the synthetic pathway by evaluating as to whether the virtual reaction on the synthetic pathway from the starting compound to the target compound that is selected in the process of S608 in FIG. 6 is attained or not. In this case, the evaluation as to whether the virtual reaction is attained or not may be done in a way that adopts a comparison between the partial structures of the compounds.

Note that the control unit 21 may omit the process of S609 in FIG. 6, may also omit the processes of S610 and S611 in FIG. 6, may further omit the processes of S612 through S614 in FIG. 6 and may still further omit the process of S615 in FIG. 6. Moreover, the control unit 21 may change the sequence of executing the process of S609 in FIG. 6, the processes of S61 0 and S611 in FIG. 6, the processes of S612 through S614 in FIG. 6 and the process of S615 in FIG. 6. For example, the control unit 21 may also, after executing the processes of S610 and S611 in FIG. 6, execute the process of S609 in FIG. 6.

Next, in a process of S616 in FIG. 6, the control unit 21 outputs items of information containing the synthetic pathway extracted in the process of S615 in FIG. 6, the respective nomenclatures of the starting compound, the intermediary compound and the target compound, which are contained in the synthetic pathway, and the enzyme used for each reaction in the synthetic pathway. In this instance, the control unit 21 specifies each reaction in the synthetic pathway extracted in the process of S615 in FIG. 6 as the known reaction or the virtual reaction, and outputs the information on the synthetic pathway extracted in the process of S615 in FIG. 6. The control unit 21 may use an enzyme EC number for the enzyme used for each reaction in the synthetic pathway.

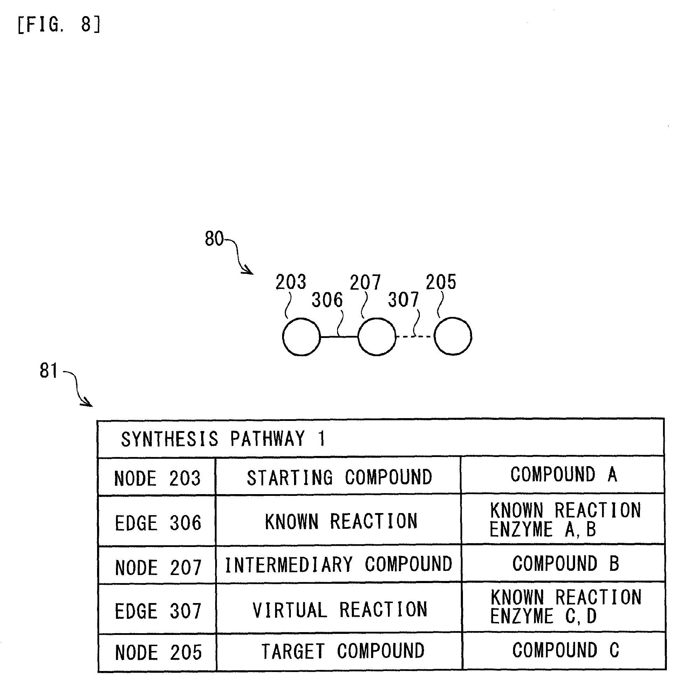

For example, the control unit 21 may output the information on the synthetic pathway extracted in the process of S615 in FIG. 6 with the aid of a graph 80 and a table 81 depicted in FIG. 8. As depicted in FIG. 8, the reaction for synthesizing the intermediary compound (compound B) from the starting compound (compound A) is specified as the known reaction, and the reaction for synthesizing the target compound (compound C) from the intermediary compound (compound B) is specified as the virtual reaction. Further, in FIG. 8, the known reaction enzymes A, B are allocated as the enzymes used for the known reaction, and the known reaction enzymes B, C are allocated as the enzymes used for the virtual reaction.

The control unit 21 may rank the synthetic pathways extracted in the process of S614 in FIG. 6 in the sequence from the largest down to the smallest of the (molar) theoretical yield (%) calculated in the process of S612 in FIG. 6. Furthermore, the control unit 21 may add, to the information that is output in the process of S616 in FIG. 6, the information on the synthetic pathways that are ranked in the sequence from the largest down to the smallest of the (molar) theoretical yield (%) calculated in the process of S612 in FIG. 6.

The control unit 21 may make a database as to the items of information that are output in the process of S616 in FIG. 6 and may store the information in the storage unit 20. Furthermore, the information, which is output in the process of S616 in FIG. 6, may also be displayed on the display unit 22.

In the present embodiment, the control unit 21 converts the chemical structures of the compounds into the UFs according to the predetermined standards, and subtracts the UF of one compound of the compound pair from the UF of the other compound, thereby calculating the DF. In the present embodiment, the control unit 21 compares the known DF of the known compound pair with the DF of the unknown compound pair and determines, as the virtual reaction, the unknown reaction of the unknown compound pair with respect to the DF coincident with the known DF of the known compound pair. In the present embodiment, the control unit 21 generates the compound synthetic pathway by the nodes indicating the respective compounds of the known compound pairs and the respective compounds of the compound pairs related to the virtual reactions and by the edges indicating the known reactions and the virtual reactions. In the present embodiment, the control unit 21 selects the synthetic pathway from the starting compound to the target compound out of the compound synthetic pathways built up by the nodes indicating the respective compounds of the known compound pairs and the respective compounds of the compound pairs related to the virtual reactions and by the edges indicating the known reactions and the virtual reactions. According to the present embodiment, a new compound synthetic pathway from the starting compound to the target compound can be selected from the compound synthetic pathways constructed by the nodes indicating the compounds and by the edges indicating the known reactions and the virtual reactions.

According to the present embodiment, the control unit 21 compares the known DF of the known compound pair with the DF of the unknown compound pair and determines, as the enzyme used for the virtual reaction, the known reaction enzyme related to the known DF of the known compound pair, which is coincident with the DF of the unknown compound pair. According to the present embodiment, it is feasible to use the known reaction enzyme for the virtual reaction contained in the selected compound synthetic pathway from the starting compound to the target compound.

### <Generation of Compound Synthetic Pathway by Optimization Method>

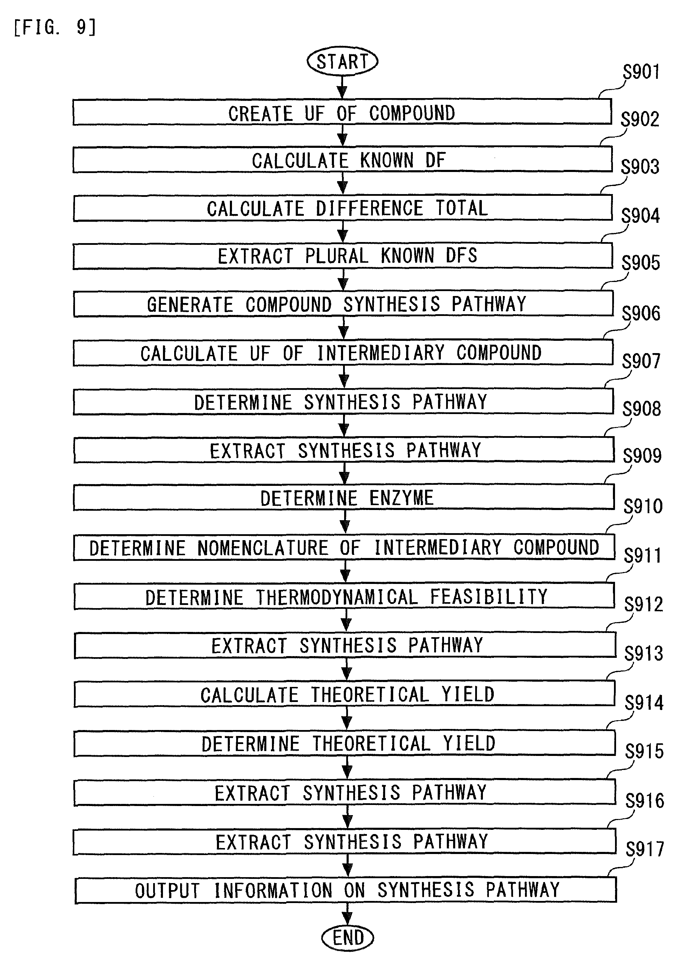

A flow of generating the compound synthetic pathway by an optimization method will be described with reference to FIG. 9. A start of the flow in FIG. 9 is triggered by accepting an instruction to generate the compound synthetic pathway by the optimization method from the user via the operation unit 10. On this occasion, designations of the starting compound and the target compound may also be accepted from the user via the operation unit 10.

In a process of S901 in FIG. 9, the control unit 21 creates the UFs of the compounds stored in the compound database 11. Namely, the control unit 21 counts the number of the partial structures contained in the chemical structural formulae of the compounds stored in the compound database 11 on the per-type basis of the partial structure, and counts the number of the bonding states of the partial structures contained in the chemical structural formulae of the compounds on the per-type basis of the partial structure and on the per-type basis of the bonding state, thereby converting the chemical structural formulae of the compounds into the numerical values. Further, in the process of S901 in FIG. 9, the control unit 21 stores the created UFs and the nomenclatures of the compounds in the UF database 15 in the way of being associated with each other. The control unit 21 executing the process of S901 in FIG. 9 functions as a converting unit.

Next, in the process of S901 in FIG. 9, the control unit 21 calculates the known DFs of the known compound pairs in the compound pairs stored in the UF database 15. The control unit 21 stores the calculated known DFs, the nomenclatures of the respective compounds of the known compound pair and the known reaction enzymes in the difference database 17 in the way of being associated with each other. The control unit 21 executing the process of S902 in FIG. 9 functions as a first calculation unit.

Then, in a process of S903 in FIG. 9, the control unit 21 calculates the difference between the UFs of the compound pair of the starting compound and the target compound by subtracting the UF of the starting compound from the UF of the target compound. In the present embodiment, the difference between the UFs of the compound pair of the starting compound and the target compound is referred to as a Difference Total. The starting compound and the target compound are designated by accepting the designations from the user via the operation unit 10. The control unit 21 executing the process of S903 in FIG. 9 functions as a second calculation unit.

Subsequently, in a process of S904 in FIG. 9, the control unit 21 extracts a plurality of known DFs from the difference database 17 so that a total value of the plural known DFs gets coincident with a value of the Difference Total by use of an integer programming algorithm. In this case, the control unit 21 may extract the plural known DFs from the difference database 17 in a way that limits the number of the known DFs. For example, the control unit 21 may extract a predetermined or smaller number of known DFs or the predetermined number of known DFs from the difference database 17. It is feasible to implement the search from within a broad range of combinations in a short period of calculation time in a way that specifies a proper condition by employing the integer programming algorithm. The control unit 21 may, however, use other methods in place of the integer programming algorithm.

The control unit 21 creates a combination (group) containing the plurality of known DFs by iterating the process of S904 in FIG. 9 a predetermined number of times. In the present embodiment, the combination containing the plurality of known DFs is termed a DF combination group. The control unit 21 creates one type of DF combination group or plural types of DF combination groups by extracting the plurality of known DFs at random from the difference database 17 each time the process of S904 in FIG. 9 is repeated.

In the process of S904 in FIG. 9, the control unit 21 extracts the plurality of known DFs from the difference database 17 and may create the DF combination group by use of the plurality of extracted known DFs. Then, the control unit 21 may also create one type of DF combination group or plural types of DF combination groups by iterating the process of S904 in FIG. 9 a predetermined number of times. In this case, the control unit 21 may extract the plurality of known DFs at random from the difference database 17 each time the process of S904 in FIG. 9 is repeated.

Next, in a process of S905 in FIG. 9, the control unit 21 generates the compound synthetic pathway from the starting compound, the target compound and the plurality of known DFs contained in the DF combination group with respect to the DF combination group generated in the process of S904 in FIG. 9. The plural types of DF combination groups are created in the process of S904 in FIG. 9, in which case control unit 21 generates the compound synthetic pathway with respect to each DF combination group. The control unit 21 executing the processes of S904 and S905 in FIG. 9 functions as a generating unit.

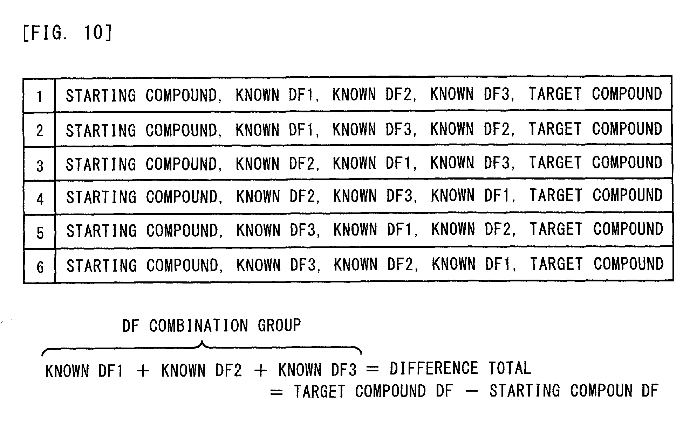

FIG. 10 illustrates examples of the compound synthetic pathways configured to include the starting compounds, the target compounds and the plurality of known DFs contained in the DF combination groups. As illustrated in FIG. 10, there are six combinations of the plural known DFs contained in the DF combination groups.

Then, in a process of S906 in FIG. 9, the control unit 21 calculates the UFs of the intermediary compounds contained in the compound synthetic pathways generated in the process of S905 in FIG. 9. The control unit 21 calculates the UFs of the intermediary compounds in the following procedures.

The compound synthetic pathway of "starting compound, known DF1, known DF2, known DF3, target compound" in the DF combination groups in FIG. 10 will be described by way of an example. Explained further is a case in which an intermediary compound A is synthesized from the starting compound, an intermediary compound B is synthesized from the intermediary compound A, and the target compound is synthesized from the intermediary compound B. To begin with, the control unit 21 calculates the UF of the intermediary compound B by subtracting the known DF3 from the UF of the target compound. Next, the control unit 21 calculates the UF of the intermediary compound A by subtracting the known DF2 from the UF of the intermediary compound B. Thus, the control unit 21 successively subtracts each of the known DFs from the UFs of the target compounds, thereby calculating the UFs of the intermediary compounds contained in the synthetic pathway of the compound, which is generated in the process of S905 in FIG. 9.

In a process of S906 in FIG. 9, the control unit 21 calculates the UFs of the intermediary compounds contained in the compound synthetic pathways generated in the process of S905 in FIG. 9 with respect to the overall combinations of the plural known DFs contained in the DF combination groups created in the process of S904 in FIG. 9. The control unit 21, if the plural types of DF combination groups are created in the process of S904 in FIG. 9, calculates the UFs of the intermediary compounds contained in the compound synthetic pathways with respect to each of the DF combination groups. The control unit 21 executing the process of S906 in FIG. 9 functions as a third calculation unit.

Subsequently, in a process of S907 in FIG. 9, the control unit 21 determines whether or not the compound synthetic pathway generated in the process of S905 in FIG. 9 is established as the synthetic pathway from the starting compound to the target compound. The control unit 21, if the synthetic pathways of the plurality of compounds are generated in the process of S905 in FIG. 9, determines whether or not each of the generated synthetic pathways is established as the synthetic pathway from the starting compound to the target compound.

An example of determining whether established as the synthetic pathway from the starting compound to the target compound or not will be described. [Determination Example 1] If the UF of the intermediary compound contained in the compound synthetic pathway generated in the process of S905 in FIG. 9 has a minus value, the control unit 21 determines that the compound synthetic pathway containing the intermediary compound is not established as the synthetic pathway from the starting compound to the target compound. If the UF of the intermediary compound has the minus value, the intermediary compound is not established as the compound, and hence the control unit 21 determines that the compound synthetic pathway containing the intermediary compound is not established as the synthetic pathway from the starting compound to the target compound.

[Determination Example 2] The control unit 21 compares atomic information of the UF of the intermediary compound contained in the compound synthetic pathway generated in the process of S905 in FIG. 9 with bond information. Then, if a discrepancy exists between the atomic information of the UF of the intermediary compound contained in the compound synthetic pathway generated in the process of S905 in FIG. 9 and the bond information, the control unit 21 determines that compound synthetic pathway containing the intermediary compound is not established as the synthetic pathway from the starting compound to the target compound. Namely, if the number of the partial structures contained in the chemical structural formula of the intermediary compound is not matched with the number of the bonding states of the partial structures contained in the chemical structural formula of the intermediary compound, the control unit 21 determines that the compound synthetic pathway containing the intermediary compound is not established as the synthetic pathway from the starting compound to the target compound.

Next, in a process of S908 in FIG. 9, the control unit 21 extracts the compound synthetic pathway determined to be established as the synthetic pathway from the starting compound to the target compound in the process of S907 in FIG. 9 out of the compound synthetic pathways generated in the process of S905 in FIG. 9.

Then, in a process of S909 in FIG. 9, the control unit 21 determines the enzymes used for synthesizing the compounds contained in the compound synthetic pathway with respect to the compound synthetic pathways extracted in the process of S908 in FIG. 9. In this case, the control unit 21 determines the enzymes used for synthesizing the compounds contained in the compound synthetic pathway by referring to the difference database 17. The difference database 17 is stored with the known DFs and the known reaction enzymes in the way of being associated with each other. Accordingly, the control unit 21 allocates the known reaction enzymes stored in the manner of being associated with the known DFs stored in the difference database 17 to the respective known DFs contained in the compound synthetic pathway extracted in the process of S908 in FIG. 9, thus determining the enzymes used for synthesizing the compounds contained in the compound synthetic pathways. The control unit 21 may allocate the plurality of known reaction enzymes stored in the difference database 17 to the respective known DFs contained in the compound synthetic pathways extracted in the process of S908 in FIG. 9.

Subsequently, in a process of S910 in FIG. 9, the control unit 21 determines the nomenclatures of the intermediary compounds contained in the compound synthetic pathways on the basis of the UFs of the intermediary compounds contained in the compound synthetic pathways with respect to the respective compound synthetic pathways extracted in the process of S908 in FIG. 9. In this case, the control unit 21 determines the nomenclatures of the intermediary compounds contained in the compound synthetic pathways by referring to the UF database 15. The control unit 21, if the UF of the intermediary compound contained in the compound synthetic pathway is coincident with the UF of the compound stored in the UF database 15, determines the nomenclature of the compound associated with the UF of the compound stored in the UF database 15 as the nomenclatures of the intermediary compound contained in the compound synthetic pathway. If the nomenclatures of the plurality of compounds are stored in the UF database 15 in the way of being associated with the UF of the compound, the control unit 21 determines the plurality of nomenclatures with respect to the intermediary compound contained in the compound synthetic pathways.

The control unit 21, if the UF of the intermediary compound contained in the compound synthetic pathway is not coincident with the UF of the compound stored in the UF database 15, does not determine the nomenclature of the intermediary compound contained in the compound synthetic pathway but allocates a predetermined number to the intermediary compound. In the present embodiment, the intermediary compound allocated with the predetermined number is termed a virtual compound without determining the nomenclature of the intermediary compound contained in the compound synthetic pathway.

For example, there is a case of storing the compound database 11 with the nomenclatures of the compounds and the data of the chemical structural formulae of the compounds, which are registered in the KEGG database, in the way of being associated with each other. In this case, the UF database 15 is stored with only the UFs of the compounds registered in the KEGG database. The UF database 15 is stored with only the UFs of the compounds registered in the KEGG database, in which case there exists a possibility that the UF of the intermediary compound contained in the compound synthetic pathway is not coincident with the UF of the compound stored in the UF database 15.

Further, in a process of S910 in FIG. 9, with respect to each of the compound synthetic pathways extracted in the process of S908 in FIG. 9, the control unit 21 determines whether each of the reactions contained in the compound synthetic pathways is the known reaction or the virtual reaction. The determination as to whether each of the reactions contained in the compound synthetic pathways is the known reaction or the virtual reaction is made in the following process.

The control unit 21 extracts, from the difference database 17, the known compound pair associated with the known DF of the intermediary compound contained in the compound synthetic pathway extracted in the process of S908 in FIG. 9. The control unit 21, if the nomenclature of each of the compounds of the extracted known compound pair is coincident with the nomenclature of each of the compounds of the compound pair contained in the compound synthetic pathway extracted in the process of S908 in FIG. 9, determines the coincident reaction of the compound pair as the known reaction. The control unit 21, whereas if the nomenclature of each of the compounds of the extracted known compound pair is not coincident with the nomenclature of each of the compounds of the compound pair contained in the compound synthetic pathway extracted in the process of S908 in FIG. 9, determines the non-coincident reaction of the compound pair as the virtual reaction. Moreover, the control unit 21, if the virtual compound is contained in the compound synthetic pathway extracted in the process of S908 in FIG. 9, determines the reaction for synthesizing another compound from the virtual compound and the reaction for synthesizing the virtual compound as the virtual reactions.

Next, in a process of S911 in FIG. 9, the control unit 21 determines the thermodynamic feasibility about the synthetic pathway to the compound extracted in the process of S908 in FIG. 9.

The determination of the thermodynamic feasibility is made by calculating a change of the free energy in each reaction contained in the compound synthetic pathway extracted in the process of S908 in FIG. 9 and determining a direction of each reaction on the basis of calculation result. If the respective reactions contained in the compound synthetic pathway extracted in the process of S908 in FIG. 9 are the forward reaction and the reversible reaction, the control unit 21 determines that there is the thermodynamic feasibility with respect to the compound synthetic pathway extracted in the process of S908 in FIG. 9. Moreover, if the reverse reaction exists in the respective reactions contained in the compound synthetic pathway extracted in the process of S908 in FIG. 9, the control unit 21 determines that there is none of the thermodynamic feasibility with respect to the compound synthetic pathway extracted in the process of S908 in FIG. 9.

Then, in a process of S912 in FIG. 9, the control unit 21 extracts the synthetic pathway having the thermodynamic feasibility out of the compound synthetic pathways extracted in the process of S908 in FIG. 9. The control unit 21 executing the processes of S911 and S912 in FIG. 9 functions as a thermodynamic determination unit.

If the virtual compound is contained in the compound synthetic pathway extracted in the process of S908 in FIG. 9, the control unit 21 executes the processes of S911 and S912 in FIG. 9 after determining the nomenclature of the virtual compound. The control unit 21 may determine the nomenclature of the virtual compound by referring to the databases provided in the devices on the external network. For instance, the UF database 15 is stored with only the UFs of the compounds registered in the KEGG database, in which case the control unit 21 may determine the nomenclatures of the virtual compounds by referring to the metabolite database such as the PubChem database, the BRENDA database and the MetaCyc database. Moreover, the control unit 21 may also determine the nomenclatures of the virtual compounds by putting a query to the user about the nomenclatures of the virtual compounds through the display unit 22 and accepting an input of the nomenclatures of the virtual compounds from the user via the operation unit 10.

The determination as to whether the compound synthetic pathway is the synthetic pathway having the thermodynamic feasibility or not entails grasping the chemical structures of the compounds. Therefore, the control unit 21 determines the nomenclatures of the compounds contained in the compound synthetic pathway extracted in the process of S908 in FIG. 9, grasps the chemical structures of the compounds from the nomenclatures of the compounds, and thus determines the synthetic pathway having the thermodynamic feasibility. The control unit 21 may also grasp the chemical structures of the compounds by referring to the compound database 11. The control unit 21 may also grasp the chemical structures of the compounds by referring to the databases provided in the devices on the external network. The control unit 21 may also grasp the chemical structures of the compounds by putting the query to the user about the chemical structures of the compounds through the display unit 22 and accepting the input of the chemical structures of the compounds from the user via the operation unit 10.

Subsequently, in a process of S913 in FIG. 9, the control unit 21 calculates the (molar) theoretical yield (%) of the target compound synthesized by the synthetic pathway extracted in the process of S912 in FIG. 9. In this case, the control unit 21 may also calculate the (molar) theoretical yield (%), derived from the raw material compound, of the target compound synthesized by the synthetic pathway extracted in the process of S912 in FIG. 9. The control unit 21, in the case of calculating the (molar) theoretical yield (%), derived from the raw material compound, of the target compound synthesized by the synthetic pathway extracted in the process of S912 in FIG. 9, refers to the known reaction database 12 and thus generates the synthetic pathway from the raw material compound to the starting compound. Then, the control unit 21 calculates the (molar) theoretical yield (%), derived from the raw material compound, of the target compound synthesized by the synthetic pathway extracted in the process of S912 in FIG. 9 by use of the synthetic pathway from the raw material compound to the starting compound and the synthetic pathway extracted in the process of S912 in FIG. 9. The raw material compound may be designated by accepting the designation from the user via the operation unit 10. In this case, the control unit 21 may generate the synthetic pathway from the raw material compound to the starting compound by referring to the data pertaining to the metabolic reaction of the *Escherichia coli*, which are stored in the known reaction database 12. The calculation of the (molar) theoretical yield can be carried out by the method described in, e.g., Biotechnology and Bioengineering, Volume 42, p.59-73 (1993).

Next, in a process of S914 in FIG. 9, the control unit 21 determines whether the (molar) theoretical yield (%) calculated in the process of S913 in FIG. 9 is equal to or larger than the threshold value or not (alternatively whether larger than the threshold value or not). The threshold value can be arbitrarily set to an arbitrary value such as 0%, 100% and 200%.

Then, in a process of S915 in FIG. 9, the control unit 21 extracts the synthetic pathway for synthesizing the target compound of which the (molar) theoretical yield (%) calculated in the process of S913 in FIG. 9 is equal to or larger than the threshold value (alternatively the (molar) theoretical yield (%) is larger than the threshold value) out of the compound synthetic pathways extracted in the process of S912 in FIG. 9. The control unit 21 executing the processes of S913, S914 and S915 in FIG. 9 functions as a (molar) theoretical yield determining unit. Even when the virtual compound is contained in the synthetic pathway of the compounds extracted in the process of S908 in FIG. 9, the control unit 21 can execute the processes of S913, S914 and S915 in FIG. 9. Even when the synthetic pathway contains a conversion reaction in which an unknown virtual compound serves as a substrate or a product, a change (an increment or a decrement in number of molecules) accompanying the conversion reaction can be grasped because of the virtual compound's UF containing numerical information of the partial structures (atoms) composing the virtual compound.

Subsequently, in a process of S916 in FIG. 9, the control unit 21 extracts the practical synthetic pathway from the synthetic pathway extracted in the process of S915 in FIG. 9. The practical synthetic pathway may be extracted in a way that evaluates the change in carbon number of each reaction that is contained in the synthetic pathway and the magnitude of the carbon number and takes account of the degree of difficulty of altering the individual virtual reaction enzymes.

Note that the control unit 21 may omit the processes of S911 and S912 in FIG. 9, may also omit the processes of S913 through S915 in FIG. 9 and may further omit the process of S916 in FIG. 9. Moreover, the control unit 21 may change the sequence of executing the processes of S911 and S912 in FIG. 9, the processes of S913 through S915 in FIG. 9 and the process of S916 in FIG. 9. For example, the control unit 21 may also, after executing the processes of S913 through S915, execute the processes of S911 and S912 in FIG. 9.

Next, in a process of S917 in FIG. 9, the control unit 21 outputs items of information containing the synthetic pathway extracted in the process of S916 in FIG. 9, the respective nomenclatures and UFs of the starting compound, the intermediary compounds and the target compound, which are contained in the synthetic pathway, and the enzymes used for each reaction in the synthetic pathway. In this instance, the control unit 21 specifies each reaction in the synthetic pathway extracted in the process of S916 in FIG.9 as the known reaction or the virtual reaction, and outputs the information on the synthetic pathway extracted in the process of S916 in FIG. 9. The control unit 21 may use the enzyme EC number for the enzyme used for each reaction in the synthetic pathway. If the virtual compound is contained in the synthetic pathway extracted in the process of S916 in FIG. 9, the control unit 21 adds, in place of the nomenclature of the intermediary compound, the predetermined number allocated to the intermediary compound to the information that is output in the process of S917 in FIG. 9.

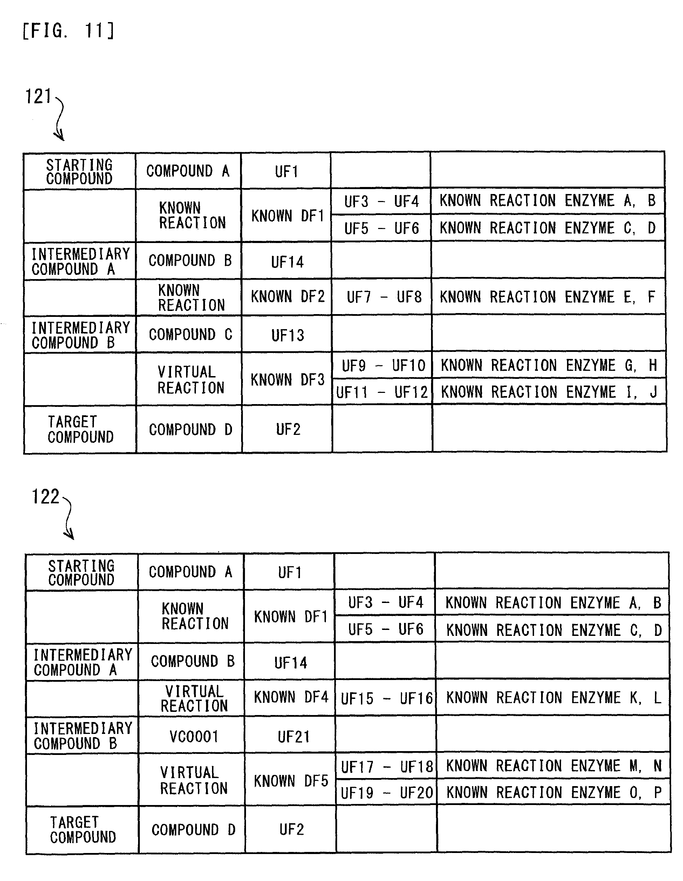

For example, the control unit 21 may output, based on Table 121 and Table 122 illustrated in FIG. 11, the synthetic pathway extracted in the process of S916 in FIG. 9, the respective nomenclatures and UFs of the starting compound, the intermediary compounds and the target compound, which are contained in the synthetic pathway, and the enzymes used for the respective reactions in the synthetic pathway. FIG. 11 illustrates an example in the case of outputting two lengths of synthetic pathways.

In Table 121 and Table 122, compounds A, B, C and D represent the nomenclatures of the compounds, and VC00001 represents the predetermined number allocated to the virtual compound. In Table 121, the reaction for synthesizing the intermediary compound A from the starting compound and the reaction for synthesizing the intermediary compound B from the intermediary compound A are specified as the known reactions. Further, in Table 121, the reaction for synthesizing the target compound from the intermediary compound B is specified as the virtual reaction. In Table 122, the reaction for synthesizing the intermediary compound A from the starting compound is specified as the known reaction. Moreover, in Table 122, the reaction for synthesizing an intermediary compound C from the intermediary compound A and the reaction for synthesizing the target compound from the intermediary compound C, are specified as the virtual reactions.

Known reaction enzymes A and B in Table 121 and Table 122 are enzymes used for the reaction of a pair of the compound specified by UF3 and the compound specified by UF4. Known reaction enzymes C and D in Table 121 and Table 122 are enzymes used for the reaction of a pair of the compound specified by UF5 and the compound specified by UF6. Known DF1 in Table 121 and Table 122 has two patterns, i.e., one pattern to take a value given by subtracting UF4 from UF3 and another pattern to take a value given by subtracting UF6 from UF5. Namely, the value given by subtracting UF4 from UF3 is the same as the value given by subtracting UF6 from UF5. Accordingly, in Table 121 and Table 122, the known reaction enzymes A, B, C and D are allocated as the enzymes used for the reaction for synthesizing the intermediary compound A from the starting compound.

Known reaction enzymes E and F in Table 121 are enzymes used for the reaction of a pair of the compound specified by UF7 and the compound specified by UF8. Known DF2 in Table 121 is a value given by subtracting UF8 from UF7. Accordingly, in Table 121, the known reaction enzymes E and F are allocated as the enzymes used for the reaction for synthesizing the intermediary compound B from the intermediary compound A.

Known reaction enzymes G and H in Table 121 are enzymes used for the reaction of a pair of the compound specified by UF9 and the compound specified by UF10. Known reaction enzymes I and J are enzymes used for the reaction of a pair of the compound specified by UF11 and the compound specified by UF12. Known DF3 in Table 121 has two patterns, i.e., one pattern to take a value given by subtracting UF10 from UF9 and another pattern to take a value given by subtracting UF12 from UF11. That is, the value given by subtracting UF10 from UF9 is the same as the value given by subtracting UF12 from UF11. Therefore, in Table 121, the known reaction enzymes G, H, I and J are allocated as the enzymes used for the reaction for synthesizing the target compound from the intermediary compound B.

Known reaction enzymes K and L in Table 122 are enzymes used for the reaction of a pair of the compound specified by UF15 and the compound specified by UF16. Known DF4 in Table 122 is a value given by subtracting UF16 from UF15. Accordingly, in Table 122, the known reaction enzymes K and L are allocated as the enzymes used for the reaction for synthesizing the intermediary compound C from the intermediary compound A.

Known reaction enzymes M and N in Table 122 are enzymes used for the reaction of a pair of the compound specified by UF17 and the compound specified by UF 18. Known reaction enzymes O and P are enzymes used for the reaction of a pair of the compound specified by UF 19 and the compound specified by UF20. Known DF5 in Table 122 has two patterns, i.e., one pattern to take a value given by subtracting UF 18 from UF 17 and another pattern to take a value given by subtracting UF20 from UF19. Therefore, in Table 122, the known reaction enzymes M, N, O and P are allocated as the enzymes used for the reaction for synthesizing the target compound from the intermediary compound C.

The control unit 21 may rank the synthetic pathways extracted in the process of S915 in FIG. 9 in the sequence from the largest down to the smallest of the (molar) theoretical yield (%) calculated in the process of S913 in FIG. 9. Furthermore, the control unit 21 may add, to the information that is output in the process of S917 in FIG. 9, the information on the synthetic pathways that are ranked in the sequence from the largest down to the smallest of the (molar) theoretical yield (%) calculated in the process of S913 in FIG. 9.

The control unit 21 may make a database as to the items of information that are output in the process of S917 in FIG. 9 and may store the information in the storage unit 20. Furthermore, the information, which is output in the process of S917 in FIG. 9, may also be displayed on the display unit 22.

In the present embodiment, the control unit 21 converts the chemical structures of the compounds into the UFs according to the predetermined standards, and calculates the DF (a first difference value) by subtracting the UF of one compound from the UF of the other compound of the compound pair. In the present embodiment, the control unit 21 calculates the DF (a second difference value) of a pair of the starting compound and the target compound by subtracting the UF of the starting compound from the UF of the target compound. In the present embodiment, the control unit 21 generates the compound synthetic pathway so that a total value of the first difference values in the synthetic pathway from the starting compound to the target compound becomes coincident with the second difference value. The compound synthetic pathway is configured to include the starting compounds, the target compounds and the plurality of first difference values.

According to the present embodiment, a new compound synthetic pathway from the starting compound to the target compound can be generated by generating the compound synthetic pathway configured to include the starting compounds, the target compounds and the plurality of first difference values. In the present embodiment, the compounds contained in the synthetic pathway from the starting compound to the target compound may be the known compounds and may also be the virtual compounds (unknown compounds). Therefore, according to the present embodiment, the synthetic pathway containing the virtual compounds can be generated. In the present embodiment, the reactions in the compound pair contained in the generated synthetic pathway from the starting compound to the target compound may be the known reactions and may also be the virtual reactions (unknown reactions). Hence, according to the present embodiment, the synthetic pathway containing the virtual reactions can be generated.

In the present embodiment, the known reaction enzyme is allocated to the first difference value contained in the generated synthetic pathway from the starting compound to the target compound. According to the present embodiment, the known reaction enzyme can be used for the virtual reaction contained in the synthetic pathway from the starting compound to the target compound.

### <Process for Producing 3-Hydroxypropionic Acid 1>

The process for producing 3-hydroxypropionic acid according to the first aspect comprises the step of reductively decarboxylating oxaloacetate to convert the oxaloacetate into 3-hydroxypropionic acid. An enzyme which reductively decarboxylates oxaloacetate to convert the oxaloacetate into 3-hydroxypropionic acid or a microorganism which has enhanced activity of such an enzyme as compared to a non-modified strain are used to carry out this step.

An example of the enzyme which reductively decarboxylates oxaloacetate to convert oxaloacetate into 3-hydroxypropionic acid includes oxaloglycolate reductase registered as EC 1.1.1.92. The activity of such an enzyme can be measured by, for example, adding oxaloglycolate or oxaloacetate as a substrate to the reaction system and observing the change in absorbance (at about 340 nm) due to the oxidation of NADH to NAD⁺ accompanied by the reduction reaction. Based on the measured activity, the enzyme can be purified from *Pseudomonas* bacteria, *Escherichia* bacteria, *Bacillus* bacteria, *Coryne*-form bacteria, yeast, and the like by a method as described in, for example, J. Biol. Chem. Vol. 243, No. 10, pp 2486-2493 (1968). A partially purified enzyme or a fraction having the enzyme activity may be used. A recombinant enzyme produced by genetic engineering may also be used.

A microorganism which has the activity of oxaloglycolate reductase can be obtained by obtaining a DNA which encodes the enzyme based on the sequence of the purified protein and introducing the DNA into a suitable host such as *Escherichia* bacteria by a method such as transformation with a vector or homologous recombination. A promoter for the gene encoding oxaloglycolate reductase on the host chromosome can also be replaced with a strong promoter to obtain a microorganism which has the activity of oxaloglycolate reductase. Microorganisms which naturally have the activity of the enzyme may also be used.

Examples of the microorganisms include but not limited to *E. coli* (*Escherichia* bacteria), *Coryne*-form bacteria, *Pseudomonas* bacteria, *Bacillus* bacteria, *Rhizobium* bacteria, *Lactobacillus* bacteria, *Succinobacillus* bacteria, *Anaerobiospirillum* bacteria, *Actinobacillus* bacteria, mold, and yeast. Treated microorganisms may also be used. Examples of the treated microorganisms include cells treated with acetone, toluene, or the like, lyophilized microorganisms, disrupted microorganisms, and cell-free extracts obtained by disrupting cells. Cells, treated cells, enzymes, and the like immobilized on a support according to a conventional procedure may also be used.

The oxaloglycolate reductase, or cells of a microorganism which has the activity of oxaloglycolate reductase or treated cells of such a microorganism can be added to a reaction liquid containing oxaloacetate and then allowed to be reacted usually at 20 to 40°C to generate 3-hydroxypropionic acid. The enzyme, however, requires NADH as a cofactor, and thus NADH should be present in the reaction system. To efficiently allow the reaction to progress, the reaction is preferably coupled with conversion of NAD⁺ into NADH. In a case where a microorganism is used, it is preferred to use a microorganism which has an enhanced activity of oxaloglycolate reductase and have the ability to convert NAD⁺ into NADH.

Microorganisms which have the ability to produce 3-hydroxypropionic acid and the activity of oxaloglycolate reductase can be used to produce 3-hydroxypropionic acid from a carbon source such as glucose by fermentation. In this case, the medium and the medium conditions for culturing the microorganisms can be determined as desired depending on the type of the microorganisms used, although a conventional medium containing a carbon source, a nitrogen source, inorganic salts, and optionally other organic trace nutrients can be used. The microorganism is cultured under conditions suitable for cell growth. Usually, the microorganisms are cultured at 10°C to 45°C for 12 to 96 hours. The term "ability to produce 3-hydroxypropionic acid" refers to the ability to accumulate 3-hydroxypropionic acid in the medium or the cells when the microorganisms are cultured in a medium containing a carbon source.

When the microorganisms are cultured, the pH is adjusted using a reagent which does not inhibit the growth of the host and which does not impede separation of 3-hydroxypropionic acid from the medium. An inorganic or organic aid or alkaline material, ammonia gas, and the like can be used to adjust the pH. Sodium carbonate, ammonia, and a sodium ion source such as, for example, sodium chloride may be added. A typical alkaline reagent such as aqueous sodium hydroxide, aqueous potassium hydroxide, aqueous ammonium hydroxide, aqueous calcium hydroxide, aqueous potassium carbonate, aqueous sodium carbonate, and aqueous potassium acetate may also be used. During culture, the pH is maintained at 5.0 or higher, preferably from 5.5 to 10.0, and more preferably 9.7 or lower.

The carbon source may be any carbon source as long as the source is assimilable by the microorganisms described above to generate 3-hydroxypropionic acid. Examples of the carbon source which can be used include fermentable carbohydrates including carbohydrates such as galactose, lactose, glucose, fructose, glycerol, sucrose, saccharose, starch, and cellulose; and polyalcohols such as glycerin, mannitol, xylitol, and ribitol.

Examples of the nitrogen source which can be used include ammonia, ammonium salts such as ammonium chloride, ammonium sulfate, and ammonium phosphate, peptone, meat extract, yeast extract, and corn steep liquor.

Examples of the inorganic salts which can be used include monopotassium phosphate, dipotassium phosphate, magnesium phosphate, magnesium sulfate, and sodium chloride.

A growth stimulating factor may be added as required, including vitamins such as biotin, pantothenic acid, inositol, and nicotinic acid, nucleotide, and amino acid.

A process for purifying 3-hydroxypropionic acid is well known in the art. For example, extraction with an organic solvent, distillation, column chromatography, or the like can be used to isolate 3-hydroxypropionic acid from the medium (US Patent No. 5,356,812). The medium may be subjected to high performance liquid chromatography (HPLC) to directly identify 3-hydroxypropionic acid.

The 3-hydroxypropionic acid obtained by the above process can be dehydrated for use in production of acrylic acid. 3-hydroxypropionic acid can be dehydrated via a known reaction. For example, as described in US Patent No. 2,469,701, 3-hydroxypropionic acid can be readily converted into acrylic acid via vacuum distillation in the presence of a catalyst.

### <Process for Producing 3-Hydroxypropionic Acid 2>

The process for producing 3-hydroxypropionic acid according to the second aspect comprises the step of reductively decarboxylating oxaloacetate to convert oxaloacetate into malonate, and the step of converting malonate into 3-hydroxypropionic acid. An enzyme which reductively decarboxylates oxaloacetate to convert oxaloacetate into malonate or a microorganism which has an enhanced activity of such an enzyme as compared to a non-modified strain is used to carry out the step of reductively decarboxylating oxaloacetate to convert oxaloacetate into malonate.

Examples of the enzyme which reductively decarboxylates oxaloacetate to convert oxaloacetate into malonate include enzymes which catalyze the reaction registered as EC 1.2.4.-. Based on the enzyme activity, such enzymes can be purified from, for example, *Pseudomonas* bacteria, *Escherichia* bacteria, *Bacillus* bacteria, *Coryne*-form bacteria, yeast, and the like. A partially purified enzyme or a fraction having the enzyme activity described above may be used. A recombinant enzyme produced by genetic engineering may also be used.

A microorganism which has the enzyme activity described above can be obtained by obtaining a DNA which encodes the enzyme described above and introducing the DNA into a suitable host such as *Escherichia* bacteria by a method such as transformation with a vector or homologous recombination. A promoter for the gene encoding the enzyme described above on the host chromosome can also be replaced with a strong promoter to obtain a microorganism which has the activity of the enzyme described above. A microorganism which naturally has the activity described above may be also used.

Any types of microorganisms may be used, including the microorganisms as described above. Treated microorganisms may also be used. Examples of the treated microorganisms include cells treated with acetone, toluene, or the like, lyophilized microorganisms, disrupted microorganisms, and cell-free extracts obtained by disrupting cells. Cells, treated cells, enzymes, and the like immobilized on a support according to a conventional procedure may also be used.

The enzyme as described above or the microorganisms which have the enzyme activity described above or treated cells of such microorganisms can be added to a reaction liquid including oxaloacetate and then allowed to be reacted usually at 20 to 40°C to generate malonate. The enzyme, however, requires NADH as a cofactor, and thus NADH should be present in the reaction system. To efficiently allow the reaction to progress, the reaction is preferably coupled with conversion of NAD⁺ into NADH. In a case where a microorganism is used, it is preferred to use a microorganism which has an enhanced activity of the enzyme described above and have the ability to convert NAD⁺ into NADH.

To carry out the step of converting malonate into 3-hydroxypropionic acid, the enzymes which catalyze the reaction registered as EC 1.2.1.15 (Biochim. Biophys. Acta. 50 (1961) 147-52.) and the enzymes which catalyze the reaction registered as EC 1.1.1.59 (J. Biol. Chem. 234 (1959) 1666-71.) can be used. These enzymes can be obtained by purification, genetic engineering, or the like. This step can be carried out usually at 20 to 40°C. The reactions catalyzed by the two enzymes may be separately performed.

Preferably, an enzyme which catalyzes the reaction registered as EC1.2.4.-, malonate-semialdehyde dehydrogenase, and 3-hydroxypropionate dehydrogenase are combined and allowed to react continuously in a single reaction system to produce 3-hydroxypropionic acid from oxaloacetate. In a case where a microorganism is used, it is preferred to use a microorganism having enhanced activity of an enzyme which catalyze the reaction registered as EC1.2.4.-, enhanced activity of malonate-semialdehyde dehydrogenase, and enhanced activity of 3-hydroxypropionate dehydrogenase.

The microorganisms which have the ability to produce 3-hydroxypropionic acid and the enzyme activity described above, and preferably the microorganisms which have the ability to produce 3-hydroxypropionic acid, enhanced activity of an enzyme which catalyze the reaction registered as EC 1.2.4.-, enhanced activity of malonate-semialdehyde dehydrogenase, and enhanced activity of 3-hydroxypropionate dehydrogenase may also be used to produce 3-hydroxypropionic acid from a carbon source such as glucose by fermentation.
The culture conditions and the process for purifying 3-hydroxypropionic acid are as described above.

### <Process for Producing 3-Hydroxypropionic Acid 3>

The process for producing 3-hydroxypropionic acid according to the third aspect comprises the step of decarboxylating pyruvate to convert pyruvate into formate and the step of converting formate into 3-hydroxypropionic acid.
To carry out the step of decarboxylating pyruvate to convert pyruvate into formate, an enzyme which decarboxylates pyruvate to convert pyruvate into formate or microorganisms which have enhanced activity of such an enzyme as compared to a non-modified strain are used.

An example of the enzyme which decarboxylates pyruvate to convert pyruvate into formate includes 2-oxoglutarate decarboxylase, which is registered as EC 4.1.1.71. The activity of this enzyme can be measured according to a method as described in, for example, FEBS Lett. 195 (1986) 43-47. Based on the measured activity, the enzyme can be purified from *Pseudomonas* bacteria, *Escherichia* bacteria, *Bacillus* bacteria, *Coryne*-form bacteria, yeast, or the like by a method as described in, for example, Arch Biochem Biophys. 288 (1991) 22-28. A partially purified enzyme or a fraction having the enzyme activity described above may be used. A recombinant enzyme produced by genetic engineering may also be used.

The microorganisms which have the enzyme activity described above can be obtained by obtaining a DNA which encodes the enzyme described above based on a known sequence and introducing the DNA into a suitable host such as *Escherichia* bacteria by a method such as transformation with a vector or homologous recombination. A promoter for the gene encoding the enzyme described above on the host chromosome can also be replaced with a strong promoter to obtain the microorganisms which have the activity of the enzyme described above. A microorganism which naturally has the activity described above may be also used.

An example of the known sequence includes but not limited to the nucleotide sequence shown in SEQ ID NO:1, which encodes 2-oxoglutarate decarboxylase of *Escherichia coli.* The known sequence may be a homolog sequence such as a DNA sequence which hybridizes, under stringent conditions, with a DNA sequence complementary to the sequence shown in SEQ ID NO:1, provided that the homolog sequence encodes a protein which has the activity of 2-oxoglutarate decarboxylase. Examples of the stringent conditions include those which are ordinary washing conditions in southern hybridization, specifically hybridization at salt concentration corresponding to 1 x SSC and 0.1% SDS, and preferably 0.1 x SSC and 0.1% SDS, at 60°C. The known sequence may be a DNA sequence which encodes a protein having an amino acid sequence at least 80%, preferably at least 90%, more preferably at least 95%, and still more preferably at least 99% identical to the amino acid sequence shown in SEQ ID NO:2 and having the activity of 2-oxoglutarate decarboxylase.

Any types of microorganisms may be used, including the microorganisms as described above. Treated microorganisms may also be used. Examples of the treated microorganisms include cells treated with acetone, toluene, or the like, lyophilized microorganisms, disrupted microorganisms, and cell-free extracts obtained by disrupting cells. Cells, treated cells, enzymes, and the like immobilized on a support according to a conventional procedure may also be used.

The enzyme as described above or the microorganisms which have the enzyme activity described above or treated cells of such microorganisms can be added to a reaction liquid including pyruvate and then allowed to be reacted usually at 20 to 40°C to generate formate.

To convert formate into 3-hydroxypropionic acid, the enzyme named malonate semialdehyde decarboxylase (EC 1.2.1.18) can be used. The enzyme is described in J Biol Chem. 2003 (49) 48674-48683 and can be obtained by purification, genetic engineering or the like. This step can be also carried out usually at 20 to 40°C.

Preferably, 2-oxoglutarate decarboxylase and malonate semialdehyde decarboxylase are combined and allowed to react continuously in a single reaction system to produce 3-hydroxypropionic acid from pyruvate. In a case where a microorganism is used, it is preferred to use the microorganism having enhanced activity of 2-oxoglutarate decarboxylase and enhanced activity of malonate semialdehyde decarboxylase.

The microorganisms which have the ability to produce 3-hydroxypropionic acid and the enzyme activity described above, and preferably the microorganisms which have the ability to produce 3-hydroxypropionic acid, enhanced activity of 2-oxoglutarate decarboxylase, and enhanced activity of malonate semialdehyde decarboxylase may also be used to produce 3-hydroxypropionic acid from a carbon source such as glucose by fermentation.
The culture conditions and the process for purifying 3-hydroxypropionic acid are as described above.

### <Process for Producing 3-Hydroxypropionic Acid 4>

The process for producing 3-hydroxypropionic acid according to the fourth aspect comprises the step of converting C02107 ((S,S)-tartaric acid) into C00222 (3-oxopropanoate) and the step of converting C00222 into 3-hydroxypropionic acid.
To carry out the step of converting C02107 ((S,S)-tartaric acid) into C00222 (3-oxopropanoate), an enzyme which converts C02107 into C00222 or microorganisms which have enhanced activity of the enzyme described above as compared to a non-modified strain are used.

An example of the enzyme which converts C02107 into C00222 includes 5-dehydro-4-deoxyglucarate dehydratase, which is registered as 4.2.1.41. The activity of this enzyme can be measured according to a method as described in, for example, Biochem. J. 115 (1969) 977-83. Based on the measured activity, the enzyme can be purified from *Pseudomonas* bacteria, *Escherichia* bacteria, *Bacillus* bacteria, *Coryne*-form bacteria, yeast, or the like by a method as described in Biochem. J. 115 (1969) 977-83. A partially purified enzyme or a fraction having the enzyme activity described above may be used. A recombinant enzyme produced by genetic engineering may also be used.

The microorganisms which have the enzyme activity described above can be obtained by obtaining a DNA which encodes the enzyme described above based on a known sequence and introducing the DNA into a suitable host such as *Escherichia* bacteria by a method such as transformation with a vector or homologous recombination. A promoter for the gene encoding the enzyme described above on the host chromosome can also be replaced with a strong promoter to obtain the microorganisms which have the activity of the enzyme described above. A microorganism which naturally has the activity described above may be also used.

An example of the known sequence includes but not limited to a sequence for 5-dehydro-4-deoxyglucarate dehydratase of *Bacillus subtilis.* An example of the sequence for the enzyme includes, for example, the nucleotide sequence shown in SEQ ID NO:3, which encodes 5-dehydro-4-deoxyglucarate dehydratase. The known sequence may be a homolog sequence such as a DNA sequence which hybridizes, under stringent conditions, with a DNA sequence complementary to the sequence shown in SEQ ID NO:3, provided that the homolog sequence encodes a protein which has the activity of 5-dehydro-4-deoxyglucarate dehydratase. Examples of the stringent conditions include those which are ordinary washing conditions in southern hybridization, specifically hybridization at salt concentration corresponding to 1 x SSC and 0.1 % SDS, and preferably 0.1 x SSC and 0.1 % SDS, at 60°C. The known sequence may be a DNA sequence which encodes a protein having an amino acid sequence at least 80%, preferably at least 90%, more preferably at least 95%, and still more preferably at least 99% identical to the amino acid sequence shown in SEQ ID NO:2 and having the activity of 5-dehydro-4-deoxyglucarate dehydratase.

Any types of microorganisms may be used, including the microorganisms as described above. Treated microorganisms may also be used. Examples of the treated microorganisms include cells treated with acetone, toluene, or the like, lyophilized microorganisms, disrupted microorganisms, and cell-free extracts obtained by disrupting cells. Cells, treated cells, enzymes, and the like immobilized on a support according to a conventional procedures may also be used.

The enzyme described above or the microorganisms which have the enzyme activity described above or treated cells of such microorganisms can be added to a reaction liquid including C02107 and then allowed to be reacted usually at 20 to 40°C to generate C00222.

To carry out the step of converting the C00222 into 3-hydroxypropionic acid, the enzyme named hydroxypropionate dehydrogenase can be used. The enzyme is described in J. Biol. Chem. 234 (1959) 1666-71 and can be obtained by purification, genetic engineering, or the like. This step can be also carried out usually at 20 to 40°C. The enzyme, however, requires NAD⁺ as a cofactor, and thus NAD⁺ should be present in the reaction system. To efficiently allow the reaction to progress, the reaction is preferably coupled with conversion of NADH into NAD⁺. In a case where a microorganism is used, it is preferred to use a microorganism which has enhanced activity of the enzyme described above and have the ability to convert NADH into NAD⁺.

Preferably, 5-dehydro-4-deoxyglucarate dehydratase and hydroxypropionate dehydrogenase are combined and allowed to react continuously in a single reaction system to produce 3-hydroxypropionic acid from C02107. In a case where a microorganism is used, it is preferred to use a microorganism having enhanced activity of 5-dehydro-4-deoxyglucarate dehydratase and enhanced activity of hydroxypropionate dehydrogenase.

The microorganisms which have the ability to produce 3-hydroxypropionic acid and the enzyme activity described above, and preferably the microorganisms which have the ability to produce 3-hydroxypropionic acid, enhanced activity of 5-dehydro-4-deoxyglucarate dehydratase, and enhanced activity of hydroxypropionate dehydrogenase may also be used to produce 3-hydroxypropionic acid from a carbon source such as glucose by fermentation.
The culture conditions and the process for purifying 3-hydroxypropionic acid are as described above.

### <Process for Producing Crotonyl Alcohol and Butadiene>

The process for producing crotonyl alcohol according to the present invention comprises the step of converting acetyl-CoA into acetoacetyl-CoA, the step of converting acetoacetyl-CoA into 3-hydroxybutyl-CoA, the step of converting 3-hydroxybutyl-CoA into crotonyl-CoA, the step of converting crotonyl-CoA into crotonyl aldehyde, and the step of converting crotonyl aldehyde into crotonyl alcohol.
To carry out the steps of converting acetyl-CoA into acetoacetyl-CoA, converting acetoacetyl-CoA into 3-hydroxybutyl-CoA, converting 3-hydroxybutyl-CoA into crotonyl-CoA, converting crotonyl-CoA into crotonyl aldehyde, and converting crotonyl aldehyde into crotonyl alcohol, enzymes which catalyze the respective conversion described above or the microorganisms which have the enzyme activities described above are used and preferably the microorganisms which have the enzyme activity to convert acetyl-CoA into acetoacetyl-CoA, the enzyme activity to convert acetoacetyl-CoA into 3-hydroxybutyl-CoA, the enzyme activity to convert 3-hydroxybutyl-CoA into crotonyl-CoA, and enhanced enzyme activity to convert crotonyl-CoA into crotonyl aldehyde and enhanced enzyme activity to convert crotonyl aldehyde into crotonyl alcohol as compared to a non-modified strain are used.

An example of the enzyme which converts acetyl-CoA into acetoacetyl-CoA includes acetyl-CoA acetyltransferase (EC1.3.1.9). An example of the enzyme which converts acetoacetyl-CoA into 3-hydroxybutyl-CoA includes 3-hydroxybutyryl-CoA dehydrogenase (1.1.1.157). An example of the enzyme which converts 3-hydroxybutyl-CoA into crotonyl-CoA includes enoyl-CoA hydratase (EC4.2.1.17).
These enzymes are found in many microorganisms. For example, 3-hydroxybutyl-CoA is known to be synthesized from *Clostridium* bacteria or the like via acetyl-CoA acetyltransferase and 3-hydroxybutyryl-CoA dehydrogenase (Youngleson, S. at el., J Bac.171:6800-6807 (1989)). It is known that a gene encoding acetyl-CoA acetyltransferase, a gene encoding 3-hydroxybutyryl-CoA dehydrogenase, and a gene encoding enoyl-CoA hydratase are present on the chromosome of *Escherichia coli,* and that activities of these enzymes are present in the bacteria (Duncombe G. et al., Arch Biochem Biophys. 176:159-170 (1976); Yang, S. and Schulz H., J. Biol. Chem. 258:9780-9785(1983); and Obrien,W., J. Bac. 132:532-540 (1977)).

Acetyl-CoA is a metabolic intermediate which is synthesized from glucose in any organisms. Thus when a host such as *Escherichia coli* is used to synthesize crotonyl alcohol, it is required to combine a gene encoding crotonyl-CoA reductase, which is the enzyme converting crotonyl-CoA into crotonyl aldehyde, and a gene encoding crotonyl alcohol hydrogenase, which is the enzyme converting crotonyl aldehyde into crotonyl alcohol, to transform the host such as *Escherichia coli.*

The specific examples of a candidate for the gene encoding crotonyl-CoA reductase and the gene encoding crotonyl alcohol dehydrogenase and a process for producing the microorganisms which use such candidates to produce crotonyl alcohol by fermentation are described below.

### <Candidate for Gene Encoding Crotonyl-CoA Reductase (Reducing Enzyme Which Produces Crotonaldehyde from Crotonyl-CoA)>

Reduction of crotonyl-CoA is catalyzed by an enzyme which reduces coenzyme A ester. An exemplary candidate for such an enzyme is cinnamoyl-CoA (having a 2-ene structure) reductase (EC 1.2.1.44) (CCR). The cinnamoyl-CoA reductase is necessary for lignin biosynthesis in higher plants, and thus such an enzyme is believed to be ubiquitously present in such plants. Two isozymes named AtCCR1 and AtCCR2 exist in *Arabidopsis thaliana.* AtCCR1 has a homology (protein sequence homology) of about 82% to AtCCR2, and each of the two isozymes is functionally expressed in *Escherichia coli* (Lauvergeat, V. Phytochemistry. 2001 57(7):1187-95). According to the result, the isozymes show different reactivity depending on the substrate. For example, AtCCR1 is about 5 times reactive toward feruloyl-CoA and sinapoyl-CoA than AtCCR2.
Similarly, a CCR protein from poplar is also expressed in *Escherichia coli* and has been studied in detail for its substrate specificity. According to Li *et al*., among the cinnamoyl-CoAs, the CCR protein from poplar has the highest affinity and the highest reaction velocity for feruloyl-CoA (Li, L. et al., Plant Cell Physiol. 2005 46(7):1073-82). The CCR has a moderate affinity for caffeoyl-CoA, although the CCR has a remarkably low maximum reaction velocity for caffeoyl-CoA. Feruloyl-CoA inhibits the CCR reaction. The CCR has a low affinity for 5-hydroxyferuloyl-CoA and sinapoyl-CoA. Thus it is apparent that cinnamoyl-CoA reductase has high substrate selectivity, although the enzyme has widely varying reactivity. Many other plants include the gene sequence encoding cinnamoyl-CoA reductase, including *Vitis vinifera, Oryza sativa japonica, Sorghum bicolor,* and *Physcomitrella patens.*

**[Table 1]**

| Gene Name | GenBank Accession No. | Source Microorganism |
|---|---|---|
| CCR1 | NC_003070.9 | *Arabidopsis thaliana* |
| CCR2 | NC_003070.9 | *Arabidopsis thaliana* |
| CCR6 | NC_008467.1 | *Populus trichocarpa* |
| CCR7 | NC_008469.1 | *Populus trichocarpa* |
| LOC100251623 | NW_002239841.1 | *Vitis vinifera* |
| Os08g0441500 | NP_001061909.1 | *Oryza sativa japonica* |
| Sb07g021680 | NC_012876.1 | *Sorghum bicolor* |
| PHYPADRAFT_111821 | NW_001865258.1 | *Physcomitrella patens subsp. patens* |

As with cinnamoyl-CoA reductase, some other enzymes catalyze redox reaction of aldehyde with coenzyme A ester. Acetaldehyde dehydrogenase (EC 1.2.1.10), which catalyzes conversion of acetaldehyde into acetyl-CoA, is involved in the metabolic pathway which converts ethyl alcohol into acetic acid. Acetaldehyde dehydrogenase of *Escherichia coli* which may be available as a host is encoded by the adhE gene and the mhpF gene. The mhpF gene forms an operon with the 3-(3-hydroxyphenyl) propionate (3-HPP) catabolic pathway genes, and is believed to catalyze the terminal reaction in the pathway.
*Pseudomonas putida* strains also contain plural acetaldehyde dehydrogenases. It is shown that the dmpF gene forms an operon with the genes encoding the proteins required for the meta-cleavage pathway for phenol degradation, and the gene is expressed in an active form in *Escherichia coli* (Powlowski J. et al., (J. Bacteriol. 1993 175, 377-385)). TodI, which forms an operon with the genes for toluene degradation, is also known (Lau, P et al., Gene, 146(1) 1994; 7-13). Mouse Aldh6A1 (Alnouti, Y and Klaassen, CD. Toxicol Sci. 2008 101(1):51-64) encoding malonate-semialdehyde dehydrogenase (EC 1.2.1.18) may also be used.

**[Table 2]**

| Gene Name | GenBank Accession No. | Source Microorganism |
|---|---|---|
| mhpF | AAC73454.1 | *Escherichia coli K-12 substr. MG1655* |
| adhE | NP_415757.1 | *Escherichia coli K-12 substr. MG1655* |
| dmpF | X60835.1 | *Pseudomonas sp. CF600* |
| todI | U09250.1 | *Pseudomonas putida F1* |
| adhE | NC_001988.2 | *Clostridium acetobutylicum ATCC 824* |
| Aldh6a1 | NC_000078.5 | *Mus musculus* |

An enzyme which catalyzes the redox reaction of a long chain aliphatic alcohol with coenzyme A ester may be useful. Some bacteria such as *Acinetobacter* are known to generate a wax ester. One of the enzymes responsible for such generation is hexadecanal dehydrogenase (acylating) (EC 1.2.1.42) encoded by the Acr1 gene (Reiser, S. and Somerville, C. J. Bacteriol. 1997 179, 2969-2975). The Acr1 gene has been cloned, and the activity has been confirmed using *Escherichia coli* as a host.

**[Table 3]**

| Gene Name | GenBank Accession No. | Source Microorganism |
|---|---|---|
| acr1 | NC_010400.1 | *Acinetobacter baumannii SDF* |
| acr1 | NC_010410.1 | *Acinetobacter baumannii AYE* |

### <Candidate for Gene Encoding Crotonyl Alcohol Dehydrogenase (Dehydrogenase Which Produces Crotonyl Alcohol from Crotonyl Aldehyde>

Reduction of crotonyl aldehyde is catalyzed by alcohol dehydrogenase (EC 1.1.1.1). Such an enzyme preferably has high reactivity toward a 2-ene-1-one structure. For example, it has been reported that the alcohol dehydrogenase encoded by the ADH1-3 gene of *Saccharomyces cerevisiae* uses 4-dimethylamino-trans-cinnamaldehyde, which is an allyl alcohol, as a substrate (Leskovac V. et al., FEMS Yeast Res. 2002 2(4):481-94). When GEDH1, which is an alcohol dehydrogenase from basil, is overexpressed in *Escherichia coli* and then the purified enzyme is analyzed, the enzyme is found to use terpene alcohols such as geraniol or cinnamyl alcohol as a substrate. Thus the enzyme can catalyze the reduction of crotonyl aldehyde using *Escherichia coli* as a host (Iijima, Y. et al., Arch Biochem Biophys.2006 448(1-2):141-149). Similarly, AtCAD5 and AtCAD4, which are cinnamyl alcohol dehydrogenase (EC 1.1.1.195) from *Arabidopsis thaliana*, have been extensively analyzed for conformation by expression in *Escherichia coli* (Youn, B. et al., Org. Biomol. Chem., 2006, 4, 1687-1697).

The alcohol dehydrogenase encoded by the Alcdh gene from *Flavobacterium* (Genbank AB084581.1) (Kazuoka, T. et al., Extremophiles. 2007 11 (2):257-67) and the alcohol dehydrogenase encoded by the ZMO1696 gene from *Zymomonas* (Kinoshita, S. et al., Appl. Microbiol. Biotechnol. 1985 22:249-254) have also been reported to oxidize allyl alcohol. Thus alcohol dehydrogenase from a procaryote may be used in the reaction described above. The alcohol dehydrogenase purified from *Escherichia coli* No. 17 strain has been reported to catalyze reaction of allyl alcohol with acrolein (Otsuka, K, J. Gen. Appl. Microbiol. 1958 4(4):211-215). Thus in a case where *Escherichia coli* is used as a host, the alcohol dehydrogenase in the host can be expected to catalyze the reaction described above.
Although the alcohol dehydrogenase from horse *(Equus caballus*) liver has not been reported to use crotonyl aldehyde as a substrate so far, the enzyme has been found to catalyze the reverse reaction in which crotonyl alcohol is oxidized to crotonyl-aldehyde (Fontaine FR et al, Chem. Res. Toxicol., 2002, 15 (8) 1051-1058). Thus it is suggested that alcohol dehydrogenase from a mammal may use crotonyl aldehyde as a substrate to catalyze a reduction reaction.

**[Table 4]**

| Gene Name | GenBank Accession No. | Source Microorganism |
|---|---|---|
| ald1 | NC_001147.5 | *Saccharomyces cerevisiae S288c* |
| ZM01696 | NC_006526.2 | *Zymomonas mobilis subsp. mobilis ZM4* |
| alcdh | AB084581.1 | *Flavobacterium frigidimaris* |
| GEDH | AY879284.1 | *Ocimum basilicum* |
| GEDH1 | AY879285.1 | *Ocimum basilicum* |
| yghD | AAC76047.1 | *Escherichia coli K-12 substr. MG1655* |
| AtCAD4 | NC_003074.8 | *Arabidopsis thaliana* |
| AtCAD5 | NC_003075.7 | *Arabidopsis thaliana* |

### <Specific Example of Production of Microorganisms Which Synthesizes Crotonyl Alcohol by Biological Method>

An *Escherichia coli* strain which has the ability to produce crotonyl alcohol can be produced using a common technique of molecular biology (Ausubel et al., Current Protocol in Molecular Biology (2003); Sambrook et al., Molecular Cloning: A Laboratory Manual, 3rd Edition (2001); and the like) and the procedure described below.
For example, the CCR1 gene from *Arabidopsis thaliana* (Genbank accession No. NC_003070.9) and the GEDH1 gene from *Ocimum basilicum* (Genbank accession No. AY879285.1) are linked together, and the linked genes are placed downstream of the lac promoter in the *Escherichia coli* plasmid vector pUC 19 (from Takara Bio Inc.) to construct the plasmid pCCR1-GEDH1. The *Escherichia coli* JM109 strain is transformed with the DNA of the constructed plasmid to produce a recombinant *Escherichia coli* strain which includes CCR1 and GEDH1. This *Escherichia coli* strain is named JM 109/pCCR1-GEDH1.

The JM 109/pCCR1-GEDH1 can be cultured by a method well known to those skilled in the art (Seibutsu Kogaku Jikkensho (2002) edited by The Society for Biotechnology, Japan; Vogel, H. et al., Fermentation and Biochemical Engineering Handbook, 2nd Ed.(2007); and the like). For example, the *Escherichia coli* JM109/pCCR1-GEDH1 can be plated on 100 ml of minimal defined medium supplemented with 20 g/L of glucose, 50 µg/ml of ampicillin, and 0.2 mM IPTG (isopropyl-β-D-thiogalactopyranoside) and cultured at 37°C for 24 hours or more using a flask. In a case where a jar fermentor is used to culture the strain, the conditions such as the substrate feed rate, stirring speed, airflow, and pH can be controlled within a proper range. An anaerobic condition, a microaerobic condition, or an aerobic condition can be selected as needed. A culturing system such as a batch culturing system, a semi culturing system, and a continuous culture can be freely selected.

The concentration of the crotonyl alcohol in the medium can be measured by high-performance liquid chromatography, gas chromatography, gas chromatography-mass spectrometry (GC-MS), liquid chromatography-mass spectrometry (LC-MS), or the like.

Expression of the CCR1 gene and the GEDH1 gene can be examined by a process well known to those skilled in the art such as northern blotting, real time PCR, and immunoblotting.

The *Escherichia coli* strain JM109/pCCR1-GEDH1 can be modified by a common molecular biologic technique (Ausubel et al., Current Protocol in Molecular Biology (2003); Sambrook et al, Molecular Cloning: A Laboratory Manual, 3rd Edition (2001); and the like) to improve the productivity of crotonyl alcohol. For example, the copy number of a gene for the rate-limiting enzyme in the crotonyl alcohol production pathway can be increased; the gene can be codon-optimized, or an amino acid in proximity to the site of the enzyme activity can be replaced to improve the enzyme activity, thereby improving the productivity of crotonyl alcohol. The productivity of crotonyl alcohol may also be improved by, for example, disrupting a gene of an enzyme catalyzing a reaction which produces by-products. A cycle of mutagenizing the strains by NTG or ultraviolet light and then selecting the strains having improved productivity of crotonyl alcohol may also be repeated.

### <Process for Producing Butadiene >

The crotonyl alcohol obtained as described above can be dehydrated to obtain butadiene. For example, Adams *et al.* has reported that butadiene is produced from crotonyl alcohol over a bismuth molybdate catalyst (Ind. Eng. Chem., 1969, 61 (6), pp 30-33). Butadiene can be produced without use of crud oil by producing crotonyl alcohol from a sugar such as glucose by fermentation using microorganisms, and then converting crotonyl alcohol, which is isolated as needed, into butadiene by the method of Adams *et al.* or the like.

Working examples will hereinafter be described. The embodiment of the present invention is not, however, limited to the following working examples.

### [Example 1]

A first working example (which will hereinafter be referred to as an Example 1) according to the present embodiment will be discussed. Example 1 is a specific example of how the compound synthetic pathway is generated based on the virtual reaction method executed by the synthetic pathway generating apparatus 1, however, numerical values according to Example 1 are exemplifications, and the present embodiment is not limited to the numerical values in Example 1.

In Example 1, the compound database 11 is stored with items of data of the nomenclatures and the chemical structural formulae of 13,213 compounds by using items of data of the nomenclatures and the chemical structural formulae of the compounds registered in the KEGG database and, in addition to the compounds registered in the KEGG database, items of data of the compound nomenclatures and the chemical structural formulae of the target compounds (including non-natural compounds). With respect to all the compounds in the KEGG database, the items of data of the nomenclatures and the chemical structural formulae of the compounds are stored in the compound database 11 by downloading KFC-formatted data notated by KEGG ATOM TYPE from the KEGG database. Data of compounds not registered in the KEGG database are converted into the KFC format by use of KegDraw tools.

Under the conditions given above, pyruvic acid is specified as the starting compound, and 3-Hydroxypropionate is specified as the target compound, in which the generation of the compound synthetic pathway is started. The control unit 21 executes the process of S601 in FIG. 6, whereby the UFs of 13,213 compounds are created and stored in the UF database 15. The control unit 21 executes the process of S602 in FIG. 6, whereby approximately 174,000,000 DFs are calculated and stored in the difference database 16.

About 134,000 unknown reactions are determined as the virtual reactions by the control unit 21 executing the processes of S603 and S604 in FIG. 6, and the control unit 21 executes the process of S605 in FIG. 6. The control unit 21 executes the process of S606 in FIG. 6, thereby creating the graph (network) containing the known reactions and the virtual reactions.

The control unit 21 carries out the process of S607 in FIG. 6, whereby the search for the generated synthetic pathway is done by the forward search algorithm in which the starting compound is defined as the starting point, and the target compound is defined as the ending point.

Under such a condition as to select the synthetic pathway from the starting compound to the target compound that involves four or less number of reaction steps, the control unit 21 executes the process of S608 in FIG. 6, thereby selecting the synthetic pathway from the starting compound to the target compound that involves four or less number of reaction steps. Under such a condition as to select the synthetic pathway from the starting compound to the target compound that involves one or less number of virtual reaction steps, the control unit 21 executes the process of S609 in FIG. 6, thereby extracting 50 compound synthetic pathways as the synthetic pathways from the starting compound to the target compound.

The control unit 21 executes the processes of S610 and S611 in FIG. 6, thereby extracting 24 compound synthetic pathways as the synthetic pathways having the thermodynamic feasibility from the 50 compound synthetic pathways. Glucose is specified as the raw material compound, and the control unit 21 executes the process of S612 in FIG. 6, thereby generating the synthetic pathways from the raw material compound to the starting compound. In this case, the control unit 21 refers to the data about the metabolic reaction of the *Escherichia coli*, which are stored in the known reaction database 12. Then, the control unit 21 executes the process of S612 in FIG. 6, thereby calculating the (molar) theoretical yield (%), derived from the raw material compound, of the target compounds synthesized by the synthetic pathways extracted in the process of S611 in FIG. 6. The glucose is specified as the raw material compound, and, under such a condition as to extract the compound synthetic pathway for synthesizing the target compound of which the (molar) theoretical yield (%) derived from the raw material compound is equal to or larger than 200%, the control unit 21 executes the processes of S613 and S614 in FIG. 6, whereby 13 compound synthetic pathways are extracted. The control unit 21 executes the process of S615 in FIG. 6, whereby 5 compound synthetic pathways are extracted as the practical synthetic pathways.

The finally extracted compound synthetic pathways contain the pathway (pyruvate → α-alanine → β-alanine → malonate semialdehyde → 3HP (3-hydroxypropionate)), which was discovered uniquely by Cargill Incorporated and disclosed in Patent document (WO/2006/022664) (refer to the following Formula 1).

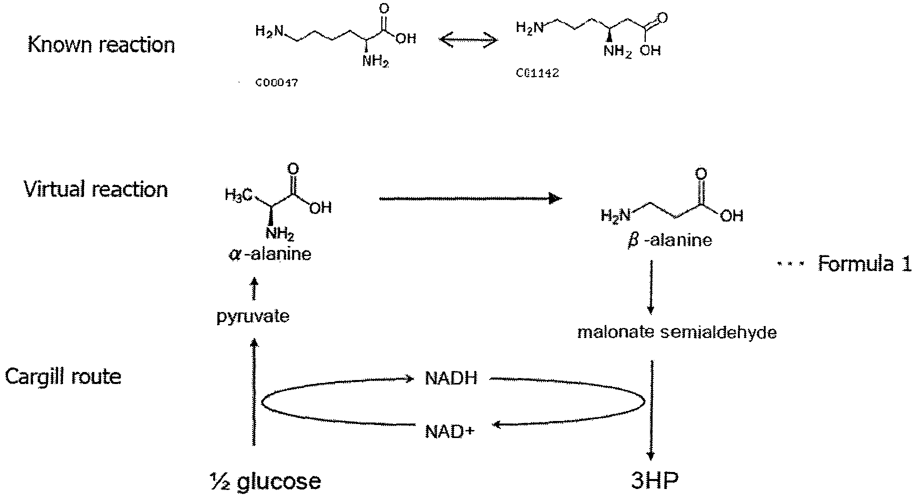

The enzyme for converting α-alanine into β-alanine is the non-natural enzyme produced by modifying lysine 2,3-aminomutase (EC 5.4.3.2) but is not registered in the KEGG database. Further, other 4 synthetic pathways, which are finally extracted, are given in the following Formulas 2 to 5.

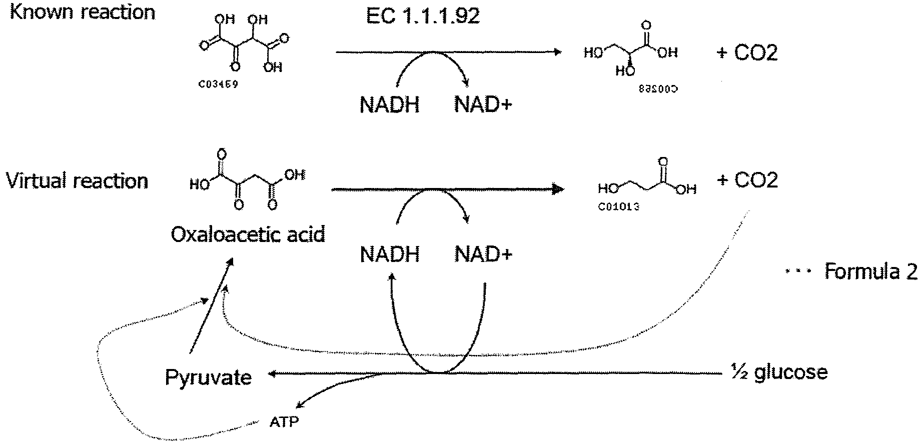

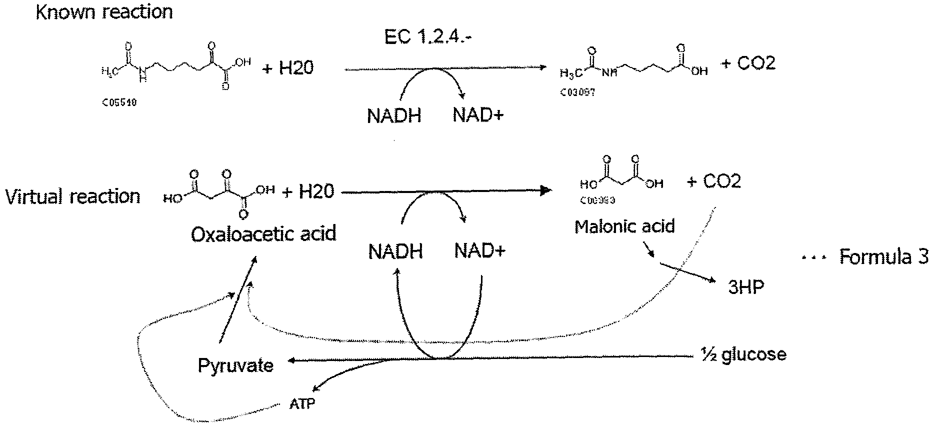

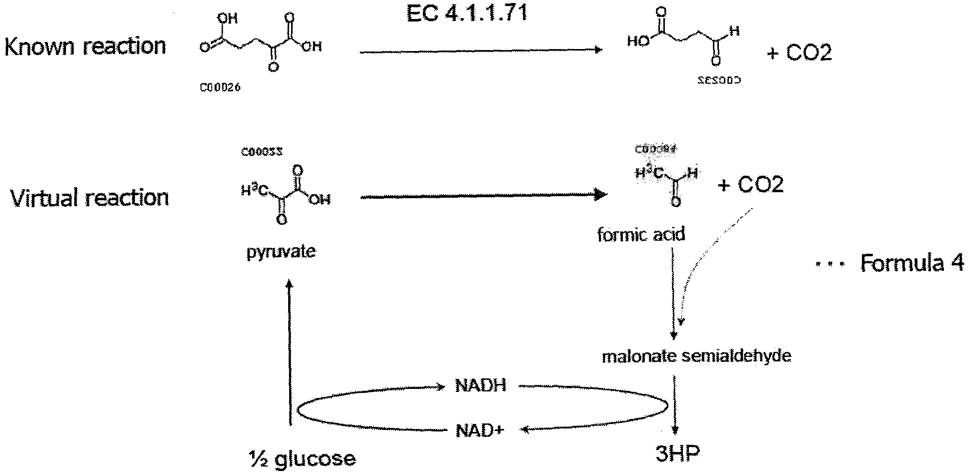

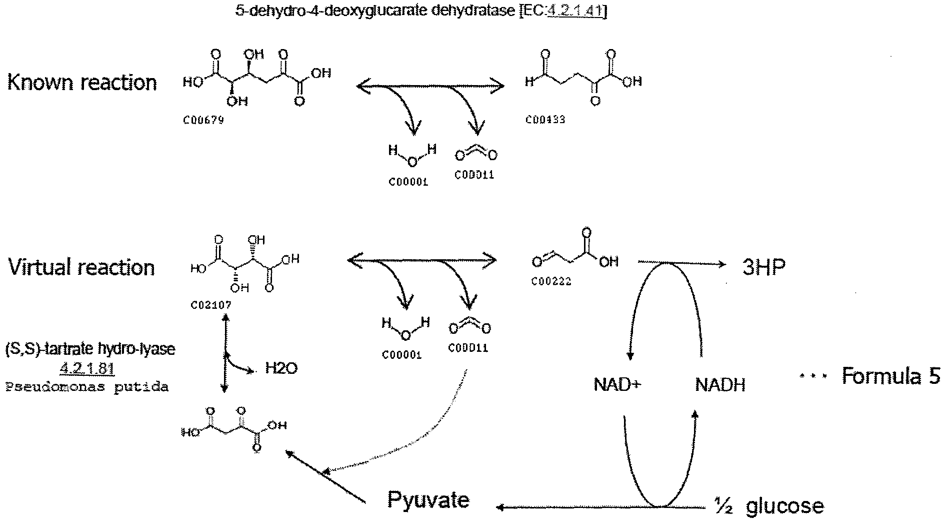

### [Example 2]

A second working example (which will hereinafter be referred to as an example 2) according to the present embodiment will be described. Example 2 is a specific example of how the compound synthetic pathway is generated based on the optimization method described above, however, the numerical values according to Example 2 are exemplifications, and the present embodiment is not limited to the numerical values in Example 2.

In Example 2, the compound database 11 is stored with the items of data of the nomenclatures and the chemical structural formulae of 13,213 compounds by using the items of data of the nomenclatures and the chemical structural formulae of the compounds registered in the KEGG database and, in addition to the compounds registered in the KEGG database, the items of data of the compound nomenclatures and the chemical structural formulae of the target compounds (including the non-natural compounds). With respect to all the compounds in the KEGG database, the items of data of the nomenclatures and the chemical structural formulae of the compounds are stored in the compound database 11 by downloading the KFC-formatted data notated by KEGG ATOM TYPE from the KEGG database. The data of compounds not registered in the KEGG database are converted into the KFC format by use of KegDraw tools.

Under the conditions given above, 4-hydroxybutyryl-CoA is specified as the starting compound, and 1,4-butanediol is specified as the target compound, in which the generation of the compound synthetic pathway is started. The control unit 21 executes the process of S901 in FIG. 9, whereby the UFs of 13,213 compounds are created and stored in the UF database 15. The control unit 21 executes the process of S902 in FIG. 9, whereby approximately 7,000 known DFs are calculated and stored in the difference database 17.

The control unit 21 executes the process of S903 in FIG. 9, whereby Difference Total of the compound pair of 4-hydroxybutyryl-CoA and 1,4-butanediol is calculated by subtracting the UF of 4-hydroxybutyryl-CoA defined as the starting compound from the UF of 1,4-butanediol defined as the target compound.

The control unit 21 executes the process of S904 in FIG. 9, thereby creating the DF combination group containing two or less number of known DFs from the difference database 17 so that the value of Difference Total is coincident with the total value of the plurality of known DFs. The control unit 21 extracts 500 known DFs at random from the difference database 17 and creates the DF combination group by use of the extracted 500 known DFs. The control unit 21 creates 6 types of DF combination groups by iterating such an operation as to extract the 500 known DFs at random from the difference database 17 and create the DF combination group by use of the extracted 500 known DFs 1,000 times.

The control unit 21 executes the process of S905 in FIG. 9, thereby creating, with respect to the 6 types of DF combination groups, 6 compound synthetic pathways from the starting compound, the target compound and the plurality of known DFs contained in the DF combination group.

The control unit 21 executes the process of S906 in FIG. 9, thereby calculating the UF of the intermediary compound contained in the compound synthetic pathway. The control unit 21 carries out the process of S907 in FIG. 9, thereby determining whether or not the 6 compound synthetic pathways are established as the synthetic pathway from the starting compound to the target compound. The control unit 21 executes the process of S908 in FIG. 9, thereby extracting the 6 compound synthetic pathways because of its being established as the synthetic pathway from the starting compound to the target compound.

The control unit 21 performs the process of S909 in FIG. 9, thereby determining, with respect to the 6 compound synthetic pathways, the enzymes used for synthesizing the compounds contained in the compound synthetic pathway. The control unit 21 carries out the process of S910 in FIG. 9, thereby determining, with respect to each of the 6 compound synthetic pathway, the nomenclature of the intermediary compound contained in the compound synthetic pathway and allocating the predetermined number to the intermediary compound of which the nomenclature cannot be determined.

The glucose is specified as the raw material compound, and the control unit 21 executes the process of S913 in FIG. 9, whereby the synthetic pathways from the raw material compound to the starting compound is generated. In this case, the control unit 21 refers to the data about the metabolic reaction of the *Escherichia coli,* which are stored in the known reaction database 12. Then, the control unit 21 executes the process of S913 in FIG. 9, thereby calculating the (molar) theoretical yield (%), derived from the raw material compound, of the target compounds synthesized by the synthetic pathways extracted in the process of 908 in FIG. 9. Subsequently, the glucose is specified as the raw material compound, and, under such a condition as to extract the compound synthetic pathway for synthesizing the target compound of which the (molar) theoretical yield (%) derived from the raw material compound is equal to or larger than 0%, the control unit 21 executes the processes of S914 and S915 in FIG. 9, whereby 5 compound synthetic pathways are extracted. Further, the glucose is specified as the raw material compound, and, under such a condition as to extract the compound synthetic pathway for synthesizing the target compound of which the (molar) theoretical yield (%) derived from the raw material compound is equal to or larger than 100%, the control unit 21 executes the processes of S914 and S915 in FIG. 9, whereby 1 compound synthetic pathway is extracted.

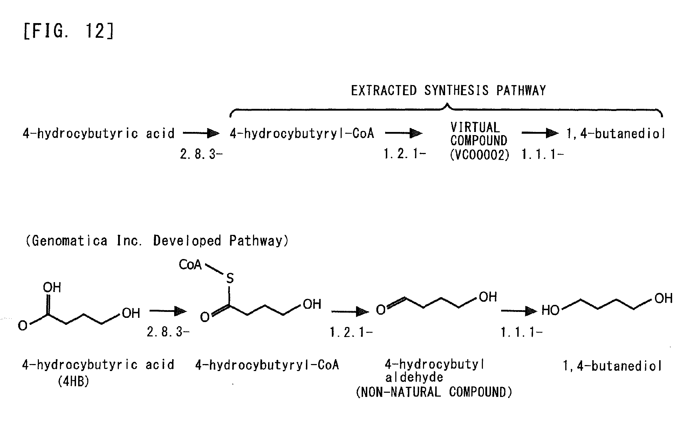

FIG. 12 depicts the finally extracted compound synthetic pathway. The finally extracted compound synthetic pathway indicates that 1,4-butanediol can be produced by consecutive reactions of three enzymes (EC 2.8.3.-, EC 1.2.1.-, EC 1.1.1.-) via 4-hydroxybutyric acid. The virtual compound contained in the finally extracted compound synthetic pathway is an intermediary compound allocated with a predetermined number (VC00002) because of being coincident with none of the UFs of the compounds stored in the UF database 15.

The finally extracted compound synthetic pathway is the same as the pathway (4-hydroxybutryl-CoA →(EC 1.2.1.-)→ 4-hydroxybutyrylaldehyde → (EC 1.2.1-)→ 1,4-butanediol) that was discovered uniquely by Genomatica Inc. (CA, USA) and disclosed in Patent document (WO/2008/115840). The optimization method involves outputting the information containing the compound synthetic pathway and the enzymes used for the respective reactions in the compound synthetic pathway, and hence the intermediary compound does not need being already known. For example, 4-hydroxybutyrylaldehyde is a compound (non-natural compound) not registered in the KEGG database, however, the synthetic pathway generated by the synthetic pathway generating apparatus 1 can contain the compounds not registered in the KEGG database. Namely, the synthetic pathway generating apparatus 1 can generate the synthetic pathway containing the compounds not registered in the KEGG database.

### [Example 3]

A third working example (which will hereinafter be referred to as Example 3) according to the present embodiment will be described. Example 3 is a specific example of how the compound synthetic pathway is generated based on the optimization method described above, however, the numerical values according to Example 3 are exemplifications, and the present embodiment is not limited to the numerical values in Example 3.

Example 3 will discuss a specific instance of generating a synthetic pathway of butadiene. In Example 3, the compound database 11 is stored with the items of data of the nomenclatures and the chemical structural formulae of 13,213 compounds by using the items of data of the nomenclatures and the chemical structural formulae of the compounds registered in the KEGG database and, in addition to the compounds registered in the KEGG database, the items of data of the compound nomenclatures and the chemical structural formulae of the target compounds (including the non-natural compounds). With respect to all the compounds in the KEGG database, the items of data of the nomenclatures and the chemical structural formulae of the compounds are stored in the compound database 11 by downloading the KFC-formatted data notated by KEGG ATOM TYPE from the KEGG database. The data of compounds not registered in the KEGG database are converted into the KFC format by use of KegDraw tools.

Under the conditions given above, acetyl-CoA is specified as the starting compound, and butadiene is specified as the target compound, in which the generation of the compound synthetic pathway is started. The control unit 21 executes the process of S901 in FIG. 9, whereby the UFs of the compounds are created and stored in the UF database 15. The control unit 21 executes the process of S902 in FIG. 9, whereby approximately 7,000 known DFs are calculated and stored in the difference database 17.

The control unit 21 executes the process of S903 in FIG. 9, whereby Difference Total of the compound pair of acetyl-CoA and butadiene is calculated by subtracting the UF of acetyl-CoA defined as the starting compound from the UF of butadiene defined as the target compound.

The control unit 21 executes the process of S904 in FIG. 9, thereby creating the DF combination group containing two or less number of known DFs from the difference database 17 so that the value of Difference Total is coincident with the total value of the plurality of known DFs. The control unit 21 extracts 500 known DFs at random from the difference database 17 and creates the DF combination group by use of the extracted 500 known DFs. The control unit 21 creates the DF combination groups by iterating such an operation as to extract the 500 known DFs at random from the difference database 17 and create the DF combination group by use of the extracted 500 known DFs 1,000 times.

The control unit 21 executes the process of S905 in FIG. 9, thereby creating, with respect to the DF combination groups, a plurality of compound synthetic pathway from the starting compound, the target compound and the plurality of known DFs contained in the DF combination groups.

The control unit 21 executes the process of S906 in FIG. 9, thereby calculating the UF of the intermediary compound contained in the compound synthetic pathway. The control unit 21 carries out the process of S907 in FIG. 9, thereby determining whether or not the plurality of compound synthetic pathway are established as the synthetic pathway from the starting compound to the target compound. The control unit 21 executes the process of S908 in FIG. 9, thereby extracting the compound synthetic pathways because of its being established as the synthetic pathway from the starting compound to the target compound.

The control unit 21 performs the process of S909 in FIG. 9, thereby determining the enzymes used for synthesizing the compounds contained in the compound synthetic pathways. The control unit 21 carries out the process of S910 in FIG. 9, thereby determining the nomenclatures of the intermediary compounds contained in the compound synthetic pathways and allocating the predetermined number to the intermediary compound of which the nomenclature cannot be determined.

The glucose is specified as the raw material compound, and the control unit 21 executes the process of S913 in FIG. 9, whereby the synthetic pathways from the raw material compound to the starting compound is generated. In this case, the control unit 21 refers to the data about the metabolic reaction of the *Escherichia coli,* which are stored in the known reaction database 12. Then, the control unit 21 executes the process of S913 in FIG. 9, thereby calculating the (molar) theoretical yield (%), derived from the raw material compound, of the target compounds synthesized by the synthetic pathways extracted in the process of 908 in FIG. 9. Subsequently, the glucose is specified as the raw material compound, and, under such a condition as to extract the compound synthetic pathway for synthesizing the target compound of which the (molar) theoretical yield (%) derived from the raw material compound is equal to or larger than 0%, the control unit 21 executes the processes of S914 and S915 in FIG. 9, whereby a compound synthetic pathway is extracted.

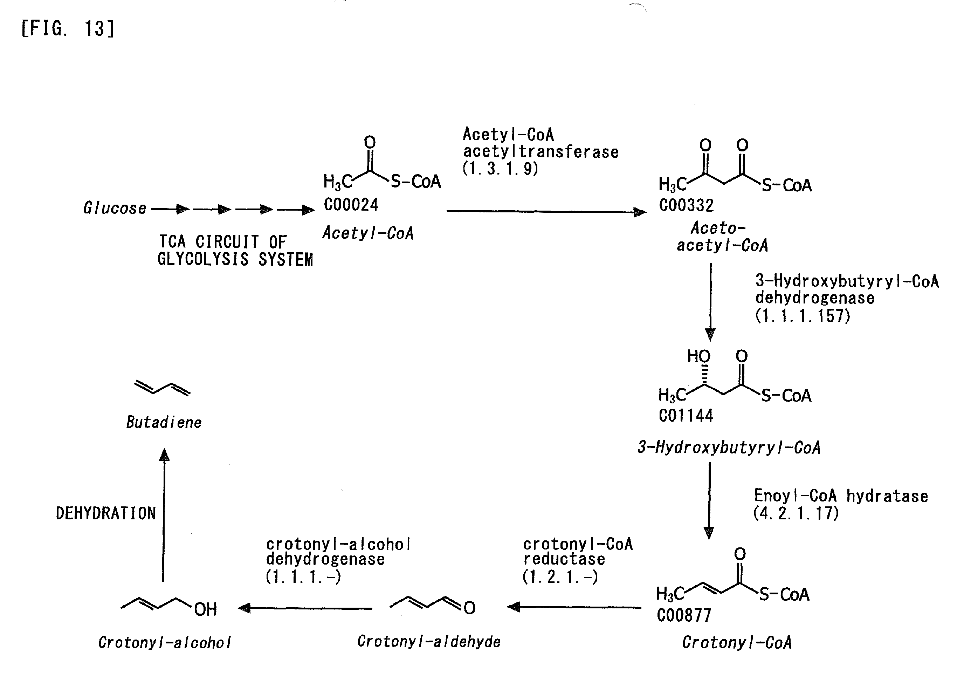

FIG. 13 depicts the finally extracted compound synthetic pathway. The finally extracted compound synthetic pathway indicates that butadiene can be, with acetyl-CoA serving as the starting compound, produced via crotonyl-CoA. The virtual compounds contained in the finally extracted compound synthetic pathway are intermediary compounds allocated with predetermined numbers because of being coincident with none of the UFs of the compounds stored in the UF database 15. Structures of these unknown intermediary substances are determined to be crotonyl aldehyde and crotonyl alcohol on the basis of the DF information.

### <Computer Readable Recording Medium>

It is possible to record a program which causes a computer to implement any of the functions described above on a computer readable recording medium. By causing the computer to read in the program from the recording medium and execute it, the function thereof can be provided. The computer readable recording medium mentioned herein indicates a recording medium which stores information such as data and a program by an electric, magnetic, optical, mechanical, or chemical operation and allows the stored information to be read from the computer. Of such recording media, those detachable from the computer include, e.g., a flexible disk, a magneto-optical disk, a CD-ROM, a CD-R/W, a DVD, a DAT, an 8-mm tape, and a memory card. Of such recording media, those fixed to the computer include a hard disk and a ROM.

### DESCRIPTION OF THE REFERENCE NUMERALS AND SYMBOLS

- 1: Synthetic pathway generating apparatus
- 2: CPU (Central Processing Unit)
- 3: Memory
- 4: Hard disk drive unit
- 5: Portable medium drive unit
- 6: Input device
- 7: Display device
- 8: External interface
- 10: Operation unit
- 11: Compound database
- 12: Known reaction database
- 13: Partial structure database
- 14: Common structure database
- 15: UF database
- 16, 17: Difference database
- 18: Virtual reaction database
- 20: Storage unit
- 21: Control unit
- 22: Display unit

## Claims

1. A synthetic pathway generating apparatus comprising:
a converting unit to convert chemical structures of compounds into numerical values;
a calculation unit to create a compound pair and to calculate a difference value by subtracting the numerical value of one compound of the compound pair from the numerical value of the other compound thereof;
a determining unit to compare the difference value of a compound pair of the reaction between which is already known with the difference value of a compound pair of the reaction between which is unknown, and to determine the unknown reaction of the compound pair with the difference value being coincident as a virtual reaction;
a generating unit to generate compound synthetic pathways by use of nodes representing the compounds and edges representing the known reaction and the virtual reaction respectively; and
a selecting unit to select a synthetic pathway from a starting compound to a target compound out of the generated compound synthetic pathways.

2. The synthetic pathway generating apparatus according to claim 1, comprising a thermodynamic determination unit to determine thermodynamic feasibility about the selected synthetic pathway.

3. The synthetic pathway generating apparatus according to claim 1 or 2, comprising a (molar) theoretical yield determining unit to calculate a (molar) theoretical yield of the target compound synthesized by the selected synthetic pathway and to determine whether or not the calculated (molar) theoretical yield is equal to or larger than a threshold value.

4. A synthetic pathway generating apparatus comprising:
a converting unit to convert chemical structures of compounds into numerical values;
a first calculation unit to create a compound pair and to calculate a first difference value by subtracting the numerical value of one compound of the compound pair from the numerical value of the other compound thereof;
a second calculation unit to calculate a second difference value by subtracting the numerical value of a starting compound from the numerical value of a target compound; and
a generating unit to generate, by use of the starting compound, the target compound and the plurality of first difference values, a synthetic pathway wherein a total value of the first difference values in the synthetic pathway from the starting compound to the target compound is coincident with the second difference value.

5. The synthetic pathway generating apparatus according to claim 4, comprising a (molar) theoretical yield determining unit to calculate a (molar) theoretical yield of the target compound synthesized by the synthetic pathway and to determine whether or not the calculated (molar) theoretical yield is equal to or larger than a threshold value.

6. The synthetic pathway generating apparatus according to claim 5, comprising a thermodynamic determination unit to determine thermodynamic feasibility about the synthetic pathway.

7. A synthetic pathway generating method by which a computer executes:
a step of converting chemical structures of compounds into numerical values;
a step of creating a compound pair and calculating a difference value by subtracting the numerical value of one compound of the compound pair from the numerical value of the other compound thereof;
a step of comparing the difference value of the compound pair of the reaction between which is already known with the difference value of the compound pair of the reaction between which is unknown, and determining the unknown reaction of the compound pair with the difference value being coincident as a virtual reaction;
a step of generating compound synthetic pathways by use of nodes representing the compounds and edges representing the known reaction and the virtual reaction respectively; and
a step of selecting a synthetic pathway from a starting compound to a target compound out of the generated compound synthetic pathways.

8. The synthetic pathway generating method according to claim 7, wherein the computer executes a step of determining thermodynamic feasibility about the selected synthetic pathway.

9. The synthetic pathway generating method according to claim 7 or 8, wherein the computer executes a step of calculating a (molar) theoretical yield of the target compound synthesized by the selected synthetic pathway and determining whether or not the calculated (molar) theoretical yield is equal to or larger than a threshold value.

10. A synthetic pathway generating method by which a computer executes:
a step of converting chemical structures of compounds into numerical values;
a step of creating a compound pair and calculating a first difference value by subtracting the numerical value of one compound of the compound pair from the numerical value of the other compound thereof;
a step of calculating a second difference value by subtracting the numerical value of a starting compound from the numerical value of a target compound; and
a step of generating, by use of the starting compound, the target compound and the plurality of first difference values, a synthetic pathway wherein a total value of the first difference values in the synthetic pathway from the starting compound to the target compound is coincident with the second difference value.

11. The synthetic pathway generating method according to claim 10, wherein the computer executes a step of calculating a (molar) theoretical yield of the target compound synthesized by the selected synthetic pathway and determining whether or not the calculated (molar) theoretical yield is equal to or larger than a threshold value.

12. The synthetic pathway generating method according to claim 11, wherein the computer executes a step of determining thermodynamic feasibility about the selected synthetic pathway.

13. A synthetic pathway generating program for making a computer execute:
a step of converting chemical structures of compounds into numerical values;
a step of creating a compound pair and calculating a difference value by subtracting the numerical value of one compound of the compound pair from the numerical value of the other compound thereof;
a step of comparing the difference value of the compound pair of the reaction between which is already known with the difference value of the compound pair of the reaction between which is unknown, and determining the unknown reaction of the compound pair with the difference values being coincident as a virtual reaction;
a step of generating compound synthetic pathways by use of nodes representing the compounds and edges representing the known reaction and the virtual reaction respectively; and
a step of selecting a synthetic pathway from a starting compound to a target compound out of the generated compound synthetic pathways.

14. The synthetic pathway generating program according to claim 13, for making the computer execute a step of determining thermodynamic feasibility about the selected synthetic pathway.

15. The synthetic pathway generating program according to claim 13 or 14, for making the computer execute a step of calculating a (molar) theoretical yield of the target compound synthesized by the selected synthetic pathway and determining whether or not the calculated (molar) theoretical yield is equal to or larger than a threshold value.

16. A synthetic pathway generating program for making a computer execute:
a step of converting chemical structures of compounds into numerical values;
a step of creating a compound pair and calculating a first difference value by subtracting the numerical value of one compound of the compound pair from the numerical value of the other compound thereof;
a step of calculating a second difference value by subtracting the numerical value of a starting compound from the numerical value of a target compound; and
a step of generating, by use of the starting compound, the target compound and the plurality of first difference values, a synthetic pathway wherein a total value of the first difference values in the synthetic pathway from the starting compound to the target compound is coincident with the second difference value.

17. The synthetic pathway generating program according to claim 16, for making the computer execute a step of calculating a (molar) theoretical yield of the target compound synthesized by the selected synthetic pathway and determining whether or not the calculated (molar) theoretical yield is equal to or larger than a threshold value.

18. The synthetic pathway generating program according to claim 17, for making the computer execute a step of determining thermodynamic feasibility about the selected synthetic pathway.

19. A method for producing 3-hydroxypropionic acid, comprising the step of reductively decarboxylating oxaloacetate to convert oxaloacetate into 3-hydroxypropionic acid.

20. A method for producing 3-hydroxypropionic acid, comprising culturing, in a medium containing a carbon source, a microorganism which has the ability to produce 3-hydroxypropionic acid and the enzyme activity to reductively decarboxylate oxaloacetate to convert oxaloacetate into 3-hydroxypropionic acid, thereby allowing to accumulate 3-hydroxypropionic acid in the medium or the cells; and collecting the 3-hydroxypropionic acid from the medium or the cells.

21. A method for producing 3-hydroxypropionic acid, comprising the step of reductively decarboxylating oxaloacetate to convert oxaloacetate into malonate; and the step of converting malonate into 3-hydroxypropionic acid.

22. A method for producing 3-hydroxypropionic acid, comprising culturing, in a medium containing a carbon source, a microorganism which has the ability to produce 3-hydroxypropionic acid, the enzyme activity to reductively decarboxylate oxaloacetate to convert oxaloacetate into malonate, and the enzyme activity to convert malonate into 3-hydroxypropionic acid, thereby allowing to accumulate 3-hydroxypropionic acid in the medium or the cells; and collecting the 3-hydroxypropionic acid from the medium or the cells.

23. A method for producing malonate, comprising the step of reductively decarboxylating oxaloacetate to convert oxaloacetate into malonate.

24. A method for producing 3-hydroxypropionic acid, comprising the step of decarboxylating pyruvate to convert pyruvate into formate; and the step of converting formate into 3-hydroxypropionic acid.

25. A method for producing 3-hydroxypropionic acid, comprising culturing, in a medium containing a carbon source, a microorganism which has the ability to produce 3-hydroxypropionic acid, the enzyme activity to decarboxylate pyruvate to convert pyruvate into formate, and the enzyme activity to convert formate into 3-hydroxypropionic acid, thereby allowing to accumulate 3-hydroxypropionic acid in the medium or the cells; and collecting the 3-hydroxypropionic acid from the medium or the cells.

26. A method for producing formate, comprising the step of decarboxylating pyruvate to convert pyruvate into formate.

27. A method for producing 3-hydroxypropionic acid, comprising the step of converting C02107 ((S,S)-tartaric acid) into C00222 (3-oxopropanoate); and the step of converting C00222 into 3-hydroxypropionic acid.

28. A method for producing 3-hydroxypropionic acid, comprising culturing, in a medium containing a carbon source, a microorganism which has the ability to produce 3-hydroxypropionic acid, the enzyme activity to convert C02107 into C00222, and the enzyme activity to convert C00222 into 3-hydroxypropionic acid, thereby allowing to accumulate 3-hydroxypropionic acid in the medium or the cells; and collecting the 3-hydroxypropionic acid from the medium or the cells.

29. A method for producing C00222, comprising the step of converting C02107 into C00222.

30. A method for producing crotonyl alcohol, comprising the step of converting acetyl-CoA into acetoacetyl-CoA; the step of converting acetoacetyl-CoA into 3-hydroxybutyl-CoA; the step of converting 3-hydroxybutyl-CoA into crotonyl-CoA; the step of converting crotonyl-CoA into crotonyl aldehyde; and the step of converting crotonyl aldehyde into crotonyl alcohol.

31. A method for producing crotonyl alcohol, comprising culturing, in a medium containing a carbon source, a microorganism which has the ability to produce crotonyl alcohol, enzyme activity to convert acetyl-CoA into acetoacetyl-CoA, the enzyme activity to convert acetoacetyl-CoA into 3-hydroxybutyl-CoA, the enzyme activity to convert 3-hydroxybutyl-CoA into crotonyl-CoA, the enzyme activity to convert crotonyl-CoA into crotonyl aldehyde, and the enzyme activity to convert crotonyl aldehyde into crotonyl alcohol, thereby allowing to accumulate crotonyl alcohol in the medium or the cells; and collecting the crotonyl alcohol from the medium or the cells.

32. A method for producing butadiene, comprising producing crotonyl alcohol by the process according to claim 30 or 31; and dehydrating the resultant crotonyl alcohol to give butadiene.
